# EUROPEAN PATENT APPLICATION

(11) **EP 4 729 600 A1**
(43) Date of publication of application: **22.04.2026**
(21) Application number: 24207762.6
(22) Date of filing: 21.10.2024
(51) Int. Cl.: C12M 1/34, C12M 1/36, G05B 13/04, G16B 40/00

(54) **COMPUTER-IMPLEMENTED METHOD FOR ESTIMATING A SPECIFIC PROTEIN PRODUCTION RATE IN BACTERIA OR YEAST**

(71) Applicant: Fermify GmbH, 1210 Vienna (AT)
(72) Inventor: Ibaceta, Maximiliano, 1210 Vienna (AT); Neudert, Mark-Richard, 1210 Vienna (AT); Herwig, Christoph, 1210 Vienna (AT)
(74) Representative: KLIMENT & HENHAPEL

(57) **Abstract**

The present invention refers to a computer-implemented method for estimating a specific protein production rate (qP) of at least one culture of protein (P) producing microorganisms in a biotechnological process, in particular a fermentation process, in a bioreactor (2), comprising at least the steps of
- obtaining data from the bioreactor (2) by measuring data and converting the measured data directly into macroscopic reaction rates to obtain online data, and/or by taking samples at least once manually or automatically from the bioreactor (2) to obtain offline data,
- combining and storing the online and/or offline data to obtain and improve a training data set;
- augmenting said training data set by processing the training data set through a mathematical model to receive estimations of specific rates, states and concentrations in the form of a time-series data,

wherein equations to describe and solve the rate transfer of carbons and electrons under the assumption of pseudo-steady state and equations to describe the development of global mass balance and total protein content over time are used by the mathematical model, wherein a probability hypothesis is generated and validated, of how the data of the training data set is interconnected, so that an augmented time-series data for the current biotechnological process, in particular the fermentation process, is produced,
- training the mathematical model with a machine learning method, using the augmented time-series data to estimate the protein production rate (qP), wherein at least one feature (Z) of the augmented time-series data is regressed against at least one of the obtained offline data of the bioreactor (2),

and the regressed time series data is used to estimate the protein production rate (qP) at any point of time of the current biotechnological process, in particular the fermentation process, as well as a system (1) for performing the computer-implemented method and a computer program product.

## Description

### FIELD OF THE INVENTION

The present invention relates to a computer-implemented method for estimating a specific protein production rate of at least one culture of protein producing microorganisms in a biotechnological process, in particular a fermentation process, in a bioreactor.

Further the invention relates to a system for performing the computer-implemented method for estimating a specific protein production rate of at least one culture of protein producing microorganisms and a computer program product comprising instructions to cause such a system perform said computer-implemented method.

### STATE OF THE ART

Production of proteins in microorganisms is well known in the prior art. In particular recombinant production of proteins in bacteria such as *Escherichia coli (E. coli)* and yeast is a main practice in both biotechnological research and pharmaceutical biotechnology due to its swift replication rates, minimal contamination risks, and comprehensive understanding of its genetics and metabolism [Chung-Jr Huang, Henry Lin, and Xiaoming Yang. "Industrial production of recombinant therapeutics in Escherichia coli and its recent advancements". In: Journal of Industrial Microbiology and Biotechnology 39.3; (Mar. 1, 2012), pp. 383-399.; Julian Kopp et al. "Repetitive Fed-Batch: A Promising Process Mode for Biomanufacturing With E. coli". In: Frontiers in Bioengineering and Biotechnology 8 (Nov. 10, 2020), p. 573607..

Beside biotechnological research and pharmaceutical biotechnology recombinant produced proteins are widely used, for example in food industry. In food industry, however, it is necessary to produce proteins highly efficient and much more cost-effective to achieve titers several orders of magnitude higher than in biotechnological research and pharmaceutical biotechnology [M.B. Nielsen, A.S. Meyer, and J. Arnau. "The Next Food Revolution Is Here: Recombinant Microbial Production of Milk and Egg Proteins by Precision Fermentation". In: Annual Review of Food Science and Technology (2023).]. Thus, already known production processes, in particular fermentation processes in a bioreactor, have to be optimized.

Within food industry it is a need to find sustainable alternatives to animal source food products, particularly for the dairy industry whose contribution to the global green house emissions is estimated at 18% [Henning Steinfeld et al. Livestock's long shadow: environmental issues and options. Food & Agriculture Org., 2006.]. Fermentation therefore is a plausible solution as an alternative for the synthesis of dairy proteins [John Puetz and Florian M. Wurm. "Recombinant Proteins for Industrial versus Pharmaceutical Purposes: A Review of Process and Pricing". In: Processes 7.8 (July 24, 2019),p. 476; Kasper Hettinga and Etske Bijl. "Can recombinant milk proteins replace those produced by animals?" In: Current Opinion in Biotechnology 75 (June 2022), p. 102690.].

In milk industry different dairy proteins are very relevant to produce different milk products. Thus, the production, in particular the recombinant production, of caseins given that 80% of the total bovine milk protein content is composed of the four caseins: αs1-casein, αs2-casein, β-casein and κ-casein, with β-casein being the most abundant, is of great interest [Kasper Hettinga and Etske Bijl. "Can recombinant milk proteins replace those produced by animals?" In: Current Opinion in Biotechnology 75 (June 2022), p. 102690.].

Different application methods for the recombinant production of β-casein in *E. coli* are widely known in the prior art. However, all of the methods are susceptible to the draw-backs and bottlenecks common to the practice of manufacturing proteins through fermentation [Chung-Jr Huang, Henry Lin, and Xiaoming Yang. "Industrial production of recombinant therapeutics in Escherichia coli and its recent advancements". In: Journal of Industrial Microbiology and Biotechnology 39.3 (Mar. 1, 2012), pp. 383-399; Zi-Xu Zhang et al. "Strategies for efcient production of recombinant proteins in Escherichia coli: alleviating the host burden and enhancing protein activity". In: Microbial Cell Factories 21.1 (Sept. 15, 2022), p. 191.]. such as the control over the formation of inclusion bodies, whose accumulation is triggered by an imbalance in the production rate and the folding and post translational modifications capabilities of the cell used for production [Arshpreet Bhatwa et al. "Challenges Associated With the Formation of Recombinant Protein Inclusion Bodies in Escherichia coli and Strategies to Address Them for Industrial Applications". In: Frontiers in Bioengineering and Biotechnology 9 (Feb. 10, 2021), p. 630551.], as well as the observed behaviour in the decay of productivity in time [Stefan Kittler et al. "Cascaded processing enables continuous upstream processing with E. coli BL21(DE3)". In: Scientific Reports 11.1 (June 1, 2021), p. 11477.]. In this regard, proper estimation, and monitoring of the specific protein productivity rate becomes a key enabler in maximizing the productivity, space time yield and titers in cultivations of bacteria, in particular *E. coli,* and yeast.

The present invention is therefore aimed at overcoming the disadvantages of the prior art.

### SUMMARY OF THE INVENTION

One object of the invention is to provide a method which enables the estimation and monitoring of specific protein production rates in cultures of bacteria or yeast, wherein it is possible to optimise the fermentation process by predicting the future production rate of the protein and by defining the best possible point in time to harvest the cells. Further, the present invention enables the estimation of other parameters used in precision fermentation for the optimization of process conditions such as fermentation time, product concentration, volumetric productivity and specific productivity.

This object is achieved by a method according to claim 1. Claim 1 contains a computer-implemented method for estimating a specific protein production rate of at least one culture of protein producing microorganisms in a biotechnological process, in particular a fermentation process in a bioreactor, comprising at least the steps of
- obtaining data from the bioreactor by measuring data and converting the measured data directly into macroscopic reaction rates to obtain online data, and/or by taking samples at least once manually or automatically from the bioreactor to obtain offline data,
- combining and storing the online and/or offline data to obtain and improve a training data set;
- augmenting said training data set by processing the training data set through a mathematical model to receive estimations of specific rates, states and concentrations in the form of a time-series data,

wherein equations to describe the rate transfer of carbons and electrons and equations to describe the development of global mass balance and total protein content over time are used by the mathematical model,
wherein a probability hypothesis is generated and validated, of how the data of the training data set is interconnected, so that an augmented time-series data for the current biotechnological process, in particular the fermentation process, is produced,
   - training the mathematical model with a machine learning method, using the augmented time-series data to estimate the protein production rate , wherein at least one feature of the augmented time-series data is regressed against at least one of the obtained offline data of the bioreactor,
and the regressed time series data is used to estimate the protein production rate at any point of time of the current biotechnological process, in particular the fermentation process.

At least one culture of microorganisms, in particular bacteria or yeast, is required to perform the biotechnological process, in particular the fermentation. Techniques for carrying out such protein production processes using microorganisms are well known in the prior art, so that it is not necessary to go into them in detail in connection with the invention. The amount of strains used depends on the size of the used bioreactor and the type of the biotechnological process. However, the present invention is not only to be used in labours, but in industry, in particular in food industry in order to be able to produce high rates of needed protein. Nonetheless, the present invention can of course used in small scale operations as well.

The microorganisms are used to produce the protein wished. Various microorganisms are used for the production of a variety proteins in prior art. As preferred microorganisms used in the present invention bacteria and yeast cells are used, however, it is of course not excluded to use other cells in the context of the present invention. The present invention generally refers to all proteins which could be produced by microorganisms in biotechnological processes.

For conducting a biotechnological process in the bioreactor an appropriate substrate, preferably for example Glycerol (also known as Glycerine) when using *E.coli*, is necessary to grow the at least one strain of microorganisms. However, other substrate can be used as well depending on the type of biotechnological process conducted. The substrate can be feed through an inlet pipe into the bioreactor. Generally it is possible to feed substrate once or constantly in the bioreactor. However, the preferred way is to add substrate if required, for example to optimize the production rate of the protein. Preferably substrate is added, when needed, as a consequence of the predictions of the protein production rate of the wished protein. It goes without saying that the substrate needed depends on the type of microorganisms used. Appropriate substrate for microorganisms used are widely known in the prior art and used accordingly. Further it is not excluded to use any helpful additives in the substrate.

As said above the present invention can be used in different biotechnological processes. One preferred way to use the present invention is in fermentation processes such as precision fermentation. The invention can in this context be used in batch, fed-batch or continuous fermentation processes. However, as those techniques are well-known to the skilled person, therefore these processes will not be discussed in detail.

Additionally, the present invention can be used in every biotechnological process, in which for example an optimization or estimation or control of a production rate or another increase or decrease rate of a cell intern rate is required. Thus, the present invention can be used for the optimization of different production processes in different industry fields.

The present invention can be used for different relevant bioprocess parameters that can be used by anyone skilled in the art for the optimization of process conditions in biotechnological processes such as: fermentation time, product concentration, volumetric productivity, specific productivity. The presented invention also establishes the baseline for a higher-tier control strategy wherein a previously unavailable estimated measurement of a property can be used for optimization of input conditions in for example fed-batch cultivations of *E. coli* that should also be evident to anyone skilled in the art of fermentation. However, different uses than the ones disclosed are not excluded.

The present invention is based on the use of online and/or offline data obtained during the biotechnological process. Further data from former or concurrently running fermentations can be used as well. The present invention can be used only with online data or only with offline data. However, the preferred embodiment uses online data as well as offline data. At least one data point each is to be used for a proper result. The more data points are used the better is the outcome.

Online data for the macroscopic reaction rates are obtained for example via softsensor which enables the conversion into the corresponding reaction rates from different data. The softsensor is specially created for the present invention in order to calculate the wished rates and states based on the initially available online data. Further the softsensor can be easily adapted to the preferred rates and states to be measured and estimated.

Offline data are generally obtained by manually or automatically taken samples from the bioreactor. The way of obtaining these data depends normally on the dimensions of the bioreactor. Offline samples are taken for example at intervals between 1,2,4 or 8 hours apart depending on the specific target of the biotechnological process. Naturally, offline samples can be taken at other intervals. However, the ways to obtain those data are known to the skilled person and it is not excluded to obtain them by other ways.

The obtained online and/or offline data are then combined and stored to obtain and improve a training data set. I.e. the data set normally is used to train the mathematical model of the present invention so that the mathematical model gets preciser the more information from different data, even from various biotechnological processes, is obtained. In case the mathematical model is used for more than one biotechnological processes like concurrently conducted fermentations estimations of later processes are then more precisely from the beginning of the process as more data can be used to estimate the production rates.

The obtained training data set is used to create a first feature matrix F, whereby initially unknown values of the feature matrix F are completed over time in course of the biotechnological process. When augmenting said training data set by processing the training data set through the mathematical model used by the present invention estimations of specific rates, states and concentrations in the form of a time-series data are obtained as a basis for further calculations. This leads to the fact that unknown points in the feature matrix F can be filled in further and further and the predictions about the production rate via rates, states and concentrations in the form of a time-series data become more and more accurate.

The mathematical basis of the mathematical model used in the present invention are equations to describe and solve the rate transfer of carbons and electrons under the assumption of pseudo-steady state and equations to describe the development of global mass balance and total protein content over time, wherein a probability hypothesis is generated and validated, of how the data of the training data set is interconnected, so that an augmented time-series data for the current fermentation is produced.

The mathematical model hereby used can be designed in different ways. One preferred possibility is that the individual processes or calculations are carried out in different blocks, whereby the number of blocks can vary as required. That means that for example the equations to describe and solve the rate transfer of carbons and electrons under the assumption of pseudo-steady state are made in one block (for example referred to as kinetic block). The equitations can be based for example on the carbon balance and the oxygen consumption in the bioreactor. However, it is also possible to base them for example on the ammonia measurement in the bioreactor.

The equations to describe the development of global mass balance and total protein content over time are made in another block (for example referred to as dynamic block), whereby the two blocks interact to generate the probability hypothesis, which is conducted by yet another block (for example referred to as state estimation block).

Based on the initially unknown model states biomass X, Protein content P, Substrate content S and Volume V of the bioreactor as well as the initially unknown parameters of the change in the substrate uptake rate qS and the growth rate µ of the biomass it is possible to estimate on specific value for every point in time on the feature matrix F for example via state estimation block. The likelihood function of the probability hypothesis is a function that determines the similitude between the plant-observation Z i.e. the offline data obtained via samples from the bioreactor and the model-computed response h, i.e. the states, rates and concentrations from the augmented training data set, derived from the interactions between the kinetic and dynamic block. In the following the mathematical model is equipped with some restrictions, especially those relating to physical, biological and chemical limits of the system, in order to limit and reduce the enormous amount of data to realistic data again.

Thus, it goes without saying that the whole mathematical model can be conducted by one block as well. Thus, it is completely variable and can be chosen and adapted by a user how the model is implemented.

In the following the mathematical model is trained with a machine learning method, using the augmented time-series data to estimate the protein production rate qP, wherein at least one feature Z of the augmented time-series data is regressed against at least one of the obtained offline data of the bioreactor. In this step it is therefore possible to compare the model-composed data with actually obtained offline data from the bioreactor. It goes without saying, that the more data are obtained the more precise are the model-composed data will get.

Lastly, the regressed time series data, i.e. the augmented time series data which has been regressed against at least one of the obtained offline data of the bioreactor, is used to estimate the protein production rate at any point of time of the current fermentation. The elements used for regression (features Z) may include various cell-specific indicators, but are not limited to: specific substrate uptake rates, qS; biomass specific inducer concentration, C_{ind}; biomass specific carbon dioxide generation rate q_{CO2}; biomass specific oxygen uptake rate, q_{O2}; respiratory quotient, R_{Q}; biomass yield on substrate, Yₓₛ, the number of generations from the culture, N_{gen}; the biomass specific growth rate µ. Inclusion of other cell-specific or culture-specific concentrations and rates relevant for anyone skilled in the art of fermentation are within the scope of this invention.

It goes without saying that the regression can of course be executed by other possible methods as well even if the result is not as proper as with the presented methods. Further it is to be said that for using the method according to the present invention comprising the mathematical model it is not mandatory that the actual relations between states and parameters are known, so that also non standard situations can be described with it or rather it can be easily adapted to such situations.

Shortly summarized the used mathematical model can be executed for example as follows: A first block contains the initial beliefs about the states x and parameters θ of the system as well as a mathematical fitting of offline protein data. The first block then interacts with a second block that yields a mass and electron balance of the biological system of the bioreactor. Therefore, the second block is compared to the plant observation for the mass and electron balance from the prior assumptions about the state and parameters and a likelihood function for each hypothesis is formulated. A third block allows then propagation of the dynamics of the system into the new time k + 1. The output of this methodology is a feature matrix F and a response variable Z which results in the specific rate of the protein production qP.

According to one embodiment of the invention the at least one culture of protein producing microorganisms are bacteria or yeast, because those microorganisms are the most common used for protein production. However, it is of course not excluded to use other microorganisms in context with the present invention.

In another embodiment of the invention the at least one culture of protein producing microorganisms are *Escherichia coli* bacteria, because those bacteria are the most well researched and relatively easy to handle. Furthermore, the processes to produce proteins via *Escherichia coli* bacteria are long-known, widely used and perfectly variable according to the individual needs. However, it is of course not excluded to use other microorganisms in context with the present invention.

In yet another embodiment of the invention the produced protein of the at least one culture of protein producing microorganisms is recombinant. The term recombinant refers to an artificially produced protein, i.e. a protein produced in a biotechnological process, which is well-known to the skilled person.

In another embodiment of the invention the produced protein is a protein used in food industry. In particular the produced protein is used to prevent the need for real animal proteins. Thus, even people with special diets can use those proteins or those protein-containing foods as provided by the present invention.

In one embodiment of the invention the produced protein is a dairy protein used in food industry. Because more people are on special diets and are looking for non-animal alternatives in foods, the protein produced is a protein used in food industry to produce alternative dairy food products.

In another embodiment of the invention the produced protein is casein protein. Casein is the name for the protein content of milk that is processed into cheese and does not end up in the whey. Casein is therefore one of the most important proteins for alternative cheese production. Therefore production of casein proteins is very relevant for food industry.

In yet another embodiment of the invention the produced protein is selected from the group comprising αS1-casein, αS2-casein, β-casein and κ-casein. Casein is a mixture of several proteins, namely αS1-casein, αS2-casein, β-casein and κ-casein. Thus, it is preferred that the produced protein is at least one of the aforementioned proteins. However, it is not excluded that more than one of the casein types are produced via the present invention.

In a further embodiment of the invention the produced protein is a protein used in biopharmaceutical industry and research. The present invention can be used to increase an optimize the production of important proteins which are needed for medications or for medicament production.

It goes without saying that the production rate of any wished protein produced by cells in any market field can be estimated and optimized by the present invention.

In one embodiment of the invention a softsensor obtains the online data referring to the percentage of oxygen O₂ and carbon dioxide CO₂ in the exhaust air of the bioreactor and converts the obtained online data into converted online data which refer to the O₂ consumption and CO₂ production in the bioreactor. As a biotechnological process is conducted in the bioreactor by aerobic microorganisms, the used microorganisms need an adapted Or content in the bioreactor to survive and produce CO₂ during their lifetime through metabolic processes. The consumed O₂ percentage and the produced CO₂ percentage in the exhaust air can be measured and and converted into various macroscopic rates, in particular CER (carbondioxide evolution rate) and OUR (oxygen uptake rate). This is particularly advantageous because the O₂ and CO₂ content can be measured relatively easily. However, it is of course not excluded to use another type of gas content for converting online data into macroscopic rates. For example the ammonia (NH₄) content for measuring the ammonia consumption in the bioreactor as an alternative or the substrate content in the bioreactor may be used. The softsensor can of course be used to obtain and measure and convert other online data as well.

In another embodiment of the invention the obtained online data are at least one selected from the group comprising Carbon Evolution Rate (CER), Oxygen Uptake Rate (OUR), ammonia uptake rate, feed rates, pH, temperature and aeration. As converted macroscopic rates the aforementioned are especially helpful, when using the O₂ content, CO₂ content or NH₄ content in the present invention. However using other online data is of course not excluded from the present invention.

In yet another embodiment of the invention the obtained offline data are at least one selected from the group comprising biomass concentration and/or mass, substrate concentration and/or mass, total protein content concentration and/or mass and/or the biomass specific concentration of protein inside the cell. Using those offline data is especially helpful, since measuring those data are well-known and relatively easy to do. However using other offline data is of course not excluded from the present invention.

The mathematical model of the present invention is based on so-called model constants, which build the backbone of the invention. Those model constants are biomass molar mass mw_{X}, substrate molar mass mw_{S}, protein molar mass such as β-casein molar mass mw_{P}, carbon dioxide molar mass mw_{CO2}, molecular oxygen molar mass mw_{O2}, biomass degree of reduction γ_{X}, substrate degree of reduction γ_{S}, protein degree of reduction such as β-casein degree of reduction γ_{P}, carbon dioxide degree of reduction γ_{CO2}, molecular oxygen degree of reduction γ_{O2}, substrate concentration Sᵢₙ in the at least one feed, specific gravity ρhcf of the at least one feed, biomass maintenance coefficient m_{c}. In one embodiment of the invention the mathematical model uses model constants, wherein the only model constants of the mathematical model that are tunable and subject to further optimization are the biomass molar mass mwₓ, the biomass maintenance coefficient m_{c} and the biomass degree of reduction γ_{X}, so that the mathematical model can be adapted for any unknown microorganisms. The fact that only these constants have to be adapted in order to be able to adapt the present invention to unknown microorganisms or rather unknown data of known microorganism shows the high flexibility and significance of the present invention.

Further the present invention is based on model states, which are the states of interest, i.e. which are the data to be obtained when using the present invention. In one further embodiment of the invention the mathematical model uses model states such as the mass of biomass X, the mass of substrate S, the relative mass of intracellular protein P and the volume V of the reactor, which model states come from previous experience and input, in order to estimate the specific protein production rate.

In another embodiment of the invention the model states are initially unknown, but the probability hypothesis h(x,θ) is formulated for every possible combination of values for initial model states x0, such as mass of biomass X, the mass of substrate S, the relative mass of intracellular protein P and the volume of the reactor V, and the parameters θ_0, such as the substrate uptake rate qS and growth rate *µ*. The mathematical model of the present invention enables to estimate the initially unknown model states based on the equitations referring to describe and solve the rate transfer of carbons and electrons under the assumption of pseudo-steady state and equations to describe the development of global mass balance and total protein content over time made together with the offline data of the bioreactor, when used. It is thus possible to calculate the likelihood of the values of the states and parameters for each point of time. Setting pre-defined limits, especially those relating to physical, biological and chemical limits of the system, lead to a very accurate estimate.

In yet another embodiment of the invention the evaluation of the probability of the true state of the bioreactor to the obtained offline data is based on sequential Monte Carlo method. The sequential Monte Carlo method or particle filter is a known method to find approximate solutions for filtering problems for nonlinear state-space systems, such as signal processing and Bayesian statistical inference. In this context the likelihood of the values of the states and parameters at one point of time can be called one particle. Particle filtering uses a set of particles to represent the posterior distribution of a stochastic process given the noisy and/or partial observations.

Thus the mathematical model of the present invention may be based on a constraint-based Bayesian augmented state and rate estimation. On basis of the estimation of the cell-specific rates and states relevant for the estimation of the production rate of the wished protein is the bayes theorem. In particular the present invention can use the Bayesian inference, an approach to statistical inference, where it is used to invert the probability of observations given a model configuration (i.e., the likelihood function) to obtain the probability of the model configuration given the observations (i.e. the posterior probability). To limit the estimation outcomes physical, biological and chemical boundaries are set, so that impossible outcomes are excluded. Further offline data can be considered when estimating the cell-specific rates and states.

In one embodiment of the invention the protein production rate qP at any point in time is formulated as *q̂_{P} = dP̂(t)*/*dt* + *µP̂* , wherein *P̂*(ₜ) is the intracellular protein in time, preferably obtained as a smoothing spline function. In this context d*P̂*(t)/dt is the rate of change of the intracellular protein in time and µ*P̂* is the product of the intracellular protein at a specific time and the growth rate obtained after evaluating the probability hypothesis. In order to achieve real-time estimation of the specific protein production rate qP [mg/gDCWh] and the intracellular protein data P [mg/gDCW] a model needs to be trained to generate an input-output response. Further the mathematical model needs to be validated as the model states and parameters have been agnostically determined.

In one further embodiment of the invention the estimated value of the production rate (qP) is formulated as a direct interaction between a smoothing spline function for the intracellular protein in time and the growth rate for each time determined. A smoothing spline function is used to compute P(t) and dP/dt from the time of induction. This leads to the fact that the estimated value of qP is formulated as a direct interaction between the smoothing spline function and the growth rate for each time determined, whereby the estimations of the protein production rate are so precise.

In another embodiment of the invention the estimated production rate qP is used to estimate the protein production and content. Since the aim of the industry is to produce as profitably as possible, the present invention can also be used to estimate protein production and content. The process can then be validated and improved according to the result.

In yet another embodiment of the invention the estimated production rate qP is used to estimate the protein production and content at any point in time in the future. Since the aim of the industry is to produce as profitably as possible, the present invention can also be used to estimate protein production and content. As a result, it is possible to intervene during the production process and make improvements or changes if necessary.

In another embodiment of the invention the time series data or rather the protein production rate qP is regressed by machine learning supervised models in the form of Partial Least Squares (PLS), Gaussian Process Regression (GPR) or Multilayer perceptron (MLP) models. Those machine learning models are well-known and perfectly adaptable for the present invention.

In yet another embodiment of the invention the augmented time series data is produced via numerical regression. This regression method is well-known and perfectly adaptable for the present invention.

The present invention further refers to a system for performing a computer-implemented method according to the claims. Thus, another object of the invention is achieved by the system according to claim 13. Claim 13 contains a system for performing a computer-implemented method according to any of claims at least comprising
- a bioreactor for performing a biotechnological process, in particular a fermentation process, comprising at least one culture of protein producing microorganisms,
- an inlet pipe for feeding at least one fluid into the bioreactor to obtain an adequate medium for the protein producing microorganisms,
- an outlet pipe for exhaust air from the bioreactor,
- an off-gas analyzer which is connected to the outlet-pipe to measure the gas content of the exhaust air,
- a mass-flow sensor which is connected to the inlet pipe to measure the mass-flow in the inlet pipe
- a dissolved oxygen sensor, which measures the dissolved oxygen in the bioreactor,
- a controlling unit to control and adapt an oxygen level in the medium of the bioreactor;
- a computing unit which is adapted to execute the computer-implemented method according to any of the claims.

The components of building the hardware of the bioreactor according to the present invention are commercially available.

At least one culture of microorganisms, in particular bacteria or yeast, is required to perform the biotechnological process. The microorganisms are to be grown in an appropriate substrate as mentioned before. The microorganisms are used to produce the protein wished. Techniques for carrying out such protein production processes using microorganisms are well known in the prior art, so that it is not necessary to go into them in detail in connection with the invention. The same applies to the used hardware.

To perform a biotechnological process the bioreactor is equipped with at least an inlet pipe and at least an outlet pipe to add or remove fluids. During the process, for example, substrate, gases or other additives can be added and, in particular, gases can be discharged. Depending on the type of biotechnological process fluids can be added or discharged once or continuously or if required. The adding or discharging can also be the result of the obtained estimated protein production rate, in order to optimize and/or increase the production rate..

The bioreactor further comprises an off-gas analyzer which is connected to the outlet-pipe to measure the gas content of the exhaust air, so that data regarding the gas content of interest can be measured. As gas of interest may be considered O₂, CO₂ or NH₄. Of course other types gas are not excluded from the present invention. Preferably softsensor obtains the data and converts them into specific states and rates.

Further the bioreactor comprises a mass-flow sensor which is connected to the inlet pipe to measure the mass-flow in the inlet pipe. Data regarding mass flow is relevant to regulate and control the fluid supply and discharge in the bioreactor.

A dissolved oxygen sensor, which measures the dissolved oxygen in the bioreactor, is also comprised to control and regulate the O₂ content in the medium. A suitable oxygen content in the substrate is required to create an ideal environment for the microorganisms so that they produce as much protein as possible and the cells are not under stress.

All sensors are generally used to control and regulate the environment in the bioreactor to create an ideal one for the microorganisms in order to maximise protein production yields. The present invention enables by estimation of the production at any point of time in future to intervene if production does not proceed as desired. In particular, this requires the further comprised Control Unit, which collects and processes the data for this purpose.

The bioreactor system is further equipped with a computing unit which is adapted to execute the computer-implemented method as described before.

In another embodiment of the invention the online and offline data are obtained by measuring and converting data from the outlet pipe. Measuring in the outlet pipe is easier to handle than in the bioreactor because the outlet pipe is easier to access.

Lastly the objective of the present invention is also reached by computer program product comprising instructions to cause a system for performing a computer-implemented method according to any of claims 1 to 12 to perform all the steps of said computer-implemented method.

Of course embodiments of the present invention comprising more components are not excluded from the scope of protections.Thus it is of course not excluded from the present invention that the bioreactor system according to the invention comprises more components for example more than one inlet pipe and more than one outlet pipe. Additional it is not excluded that the system only comprises one pipe which serves then as inlet and outlet pipe. I.e. the bioreactor can be more compact or larger depending on the needs of the user.

In order to better illustrate the present invention, some examples are set out below.

### Example 1:

In example 1 a fed-batch fermentation is conducted in a bioreactor using strains of *Escherichia coli* bacteria. As substrate S Glycerol was used. Offline data were manually taken from the bioreactor every 2 hours starting from the moment of induction and are presented in Table 1. Every 2 hours biomass X, Glycerol content S (also referred to as G in the diagram), protein content P and volume V of the bioreactor as model states i.e. the variables of interest, were determined.

**Table 1: Offline sample time series for state augmentation**

| Time | Biomass [g/L] | Glycerin [g/L] | Protein [mg/gDCW] | Volume [1] |
|---|---|---|---|---|
| 15.5 | 7.3 | N.D | 0 | 1.091 |
| 17.5 | 11.1 | N.D | 29.72 | 1.086 |
| 19.5 | 14.1 | N.D | 65.26 | 1.093 |
| 22 | 18.8 | N.D | 82.93 | 1.127 |
| 24 | 23.9 | N.D | 67.90 | 1.173 |

The online data obtained for this fermentation available is disclosed in Diagram 2. The online data were obtained via off gas analyzer mounted in an outlet pipe of the bioreactor, whereby the offgas analyzer measured the O₂ and CO₂ content of the exhaust gas. The measured O₂ and CO₂ content data from the offgas analyzers where then converted by a softsensor into CER and OUR values. Further the feed rate of the substrate, Glycerol G, into the bioreactor was obtained by measuring and converting the mass flow in an inlet pipe via mass flow sensor.

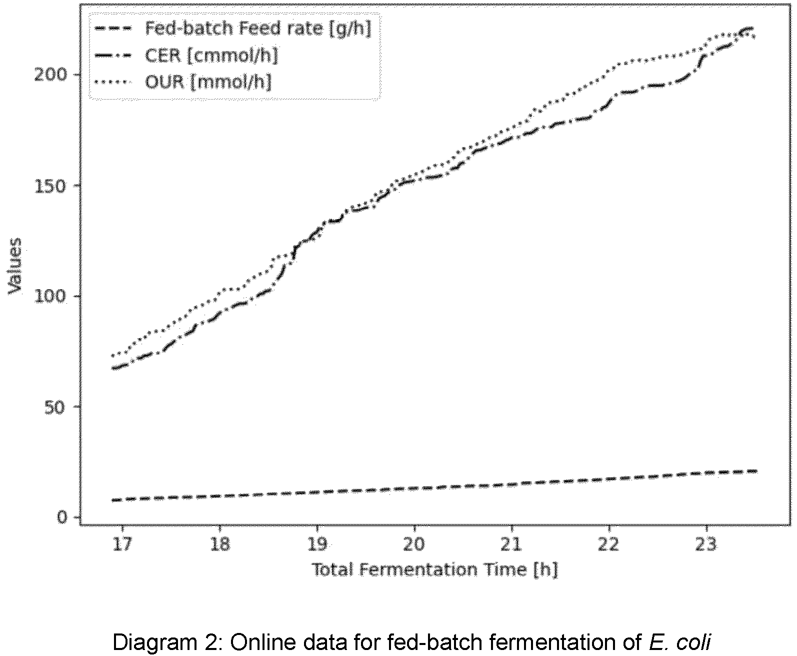

The online and offline date were then stored and combined to receive the training data set.

Following the training data set was augmented by processing the training data set through the
mathematical model to receive estimations of specific rates, states and concentrations in the form of a time-series data. In the present example this is made via a smoothing spine equitation. The smoothing spline fit for the datapoints in P_{off}, yields information about a potential trajectory for P in time and also, in accordance with values for the time derivative of the intracellular protein which has been calculated via the mathematical model as observed in Diagram 3.

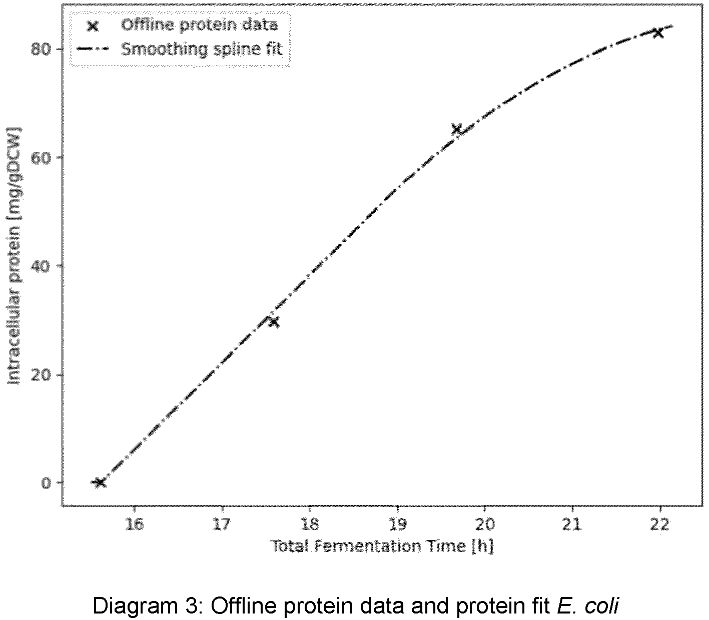

Using the mathematical model of the present invention a probability hypothesis is generated and validated, of how the data of the training data set is interconnected, so that an augmented time-series data for the the current fed-batch fermentation is produced. Therefore the mathematical model was trained with a machine learning method, using the augmented time-series data to estimate the protein production rate qP, wherein at least one feature Z of the augmented time-series data is regressed against at least one of the obtained offline data of the bioreactor. Therefore the particle filter estimations i.e. the hypothesis for any possible combination at one point in time, were performed every 100 seconds from the moment of induction and the surviving particles were propagated using the smoothing spline input for protein P and the rate of change of intracellular protein in time (dP/dt).

Afterwards the regressed time series data is used to estimate the protein production rate qP at any point of time of the current biotechnological process, in particular the fermentation. Therefore the results were then plotted and a R2 score of 0.90 was reached. The reconstructed online data points are presented in Diagram 4.

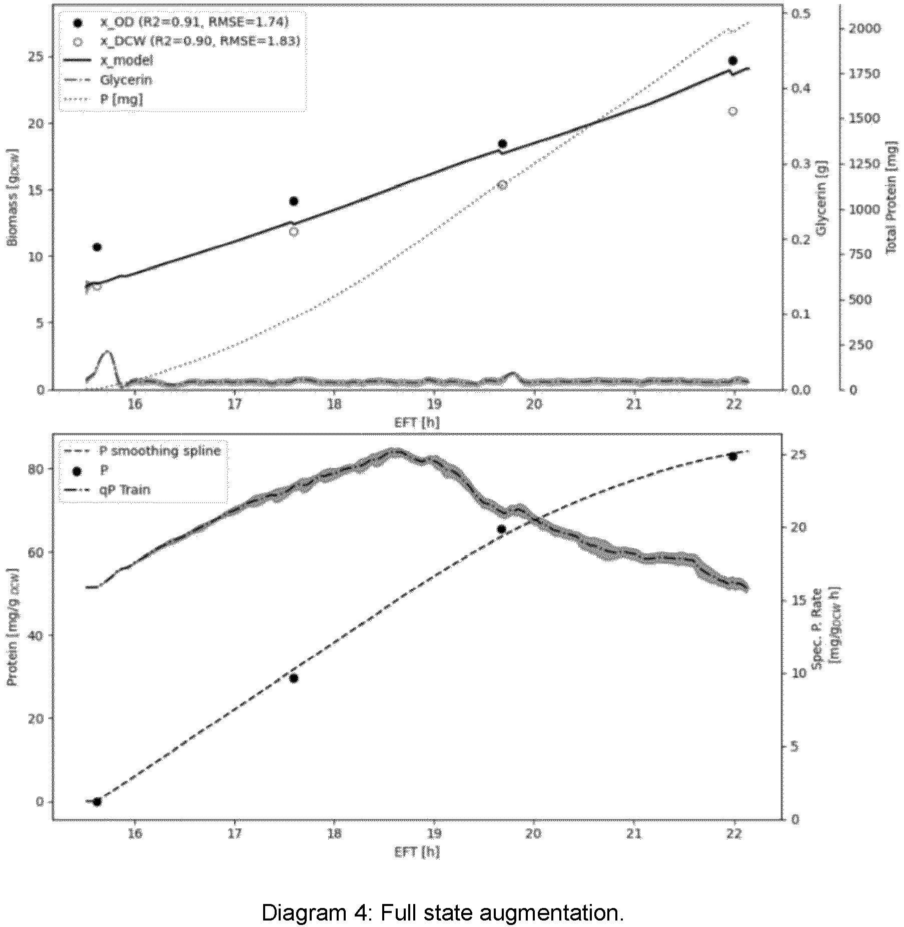

Furthermore, the reconstruction of all the biomass specific rates, as well as the relationship between relevant features Z and qP are plotted in Diagram 5. Diagram 5 consists of eleven Sub-Diagrams 5a to 5k.

The first row of Diagram 5 shows data referring to Biomass X, Protein P, Substrate G and the volume V of the bioreactor as offline data as measured and as estimated (Sub-diagrams 5a-5c). The second row of Diagram 5 shows CER, specific rates (protein production rate qP and substrate uptake rate qS/qG) and growth rates as online data and augmented times series data as measured and estimated (Sub-diagrams 5d-5f). The third and forth row each shows the protein production rate over time in different diagrams as measured and as expected (Sub-diagrams 5g-5k). In the following the Sub-diagrams are disclosed, in order to be able to better visualise the content.

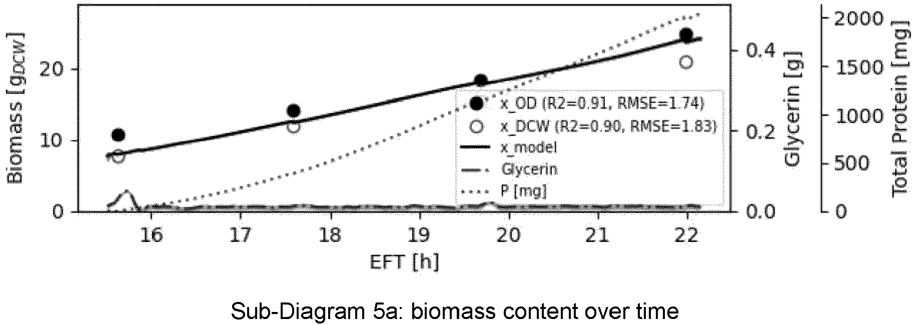

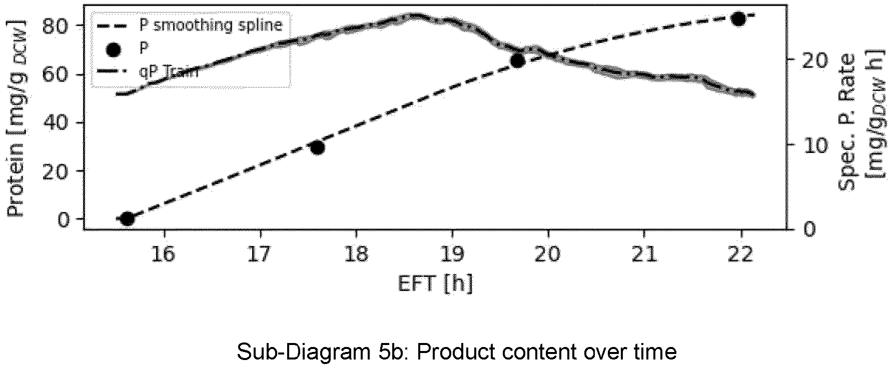

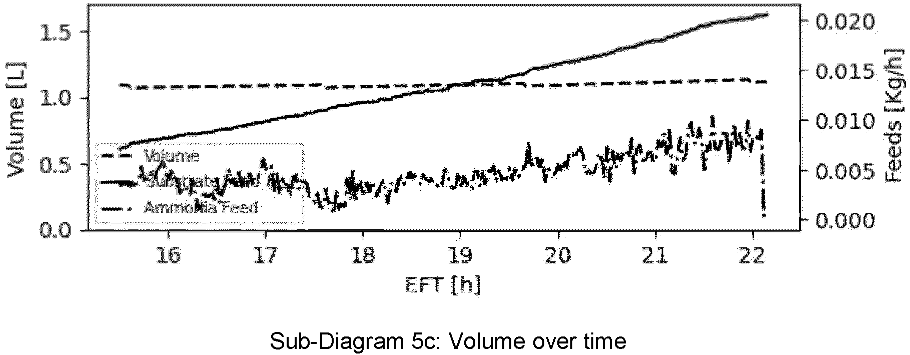

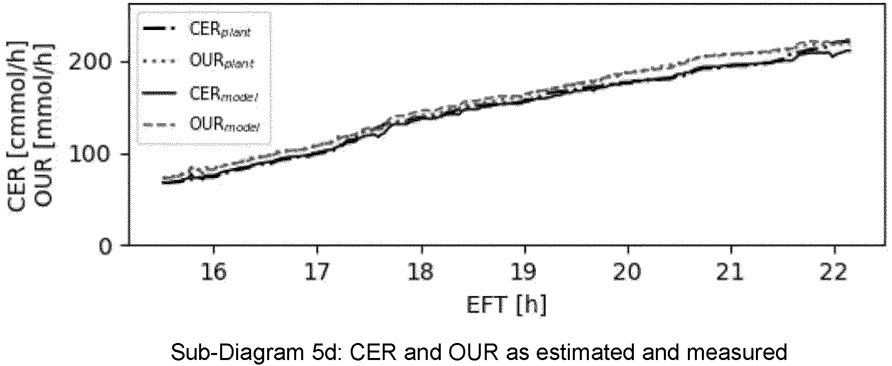

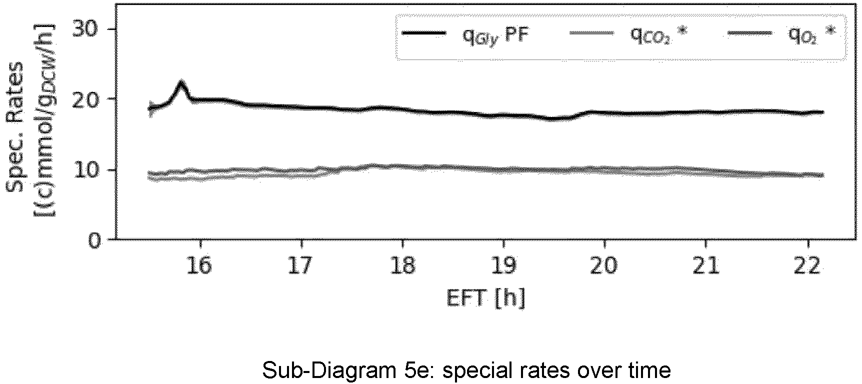

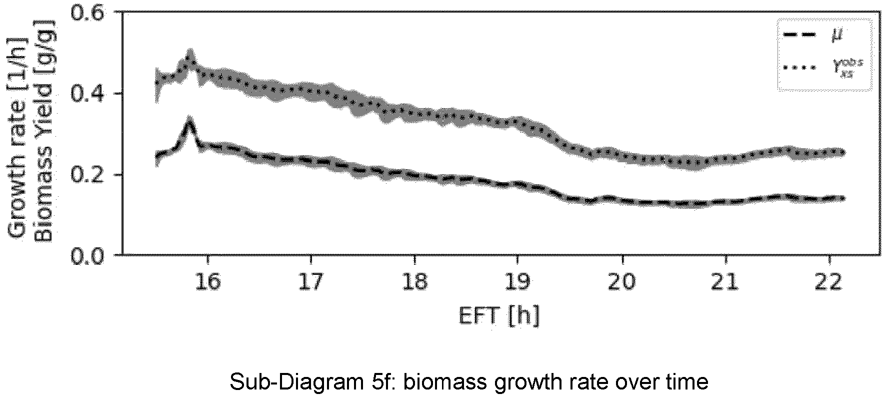

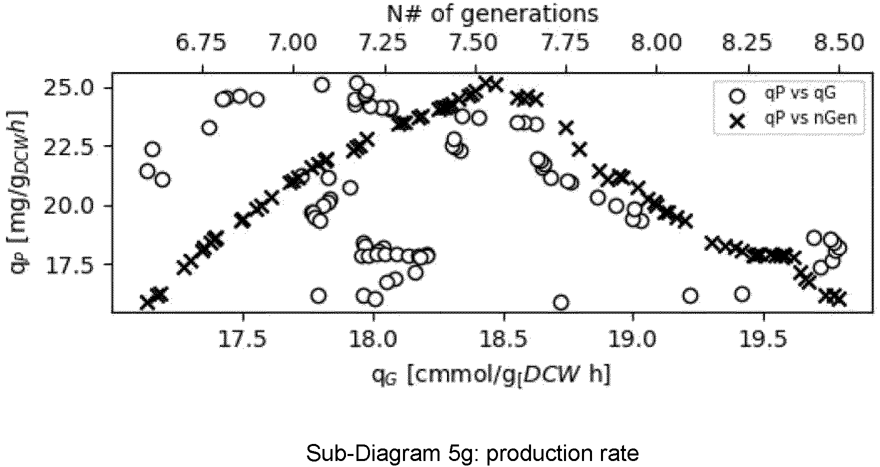

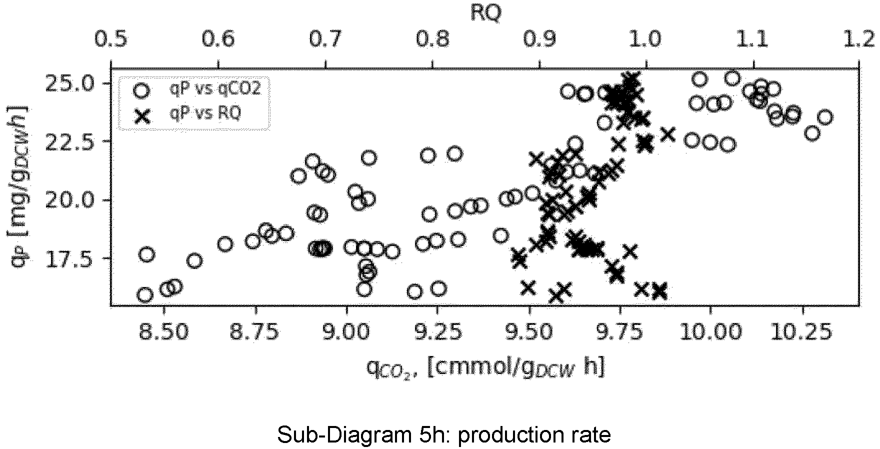

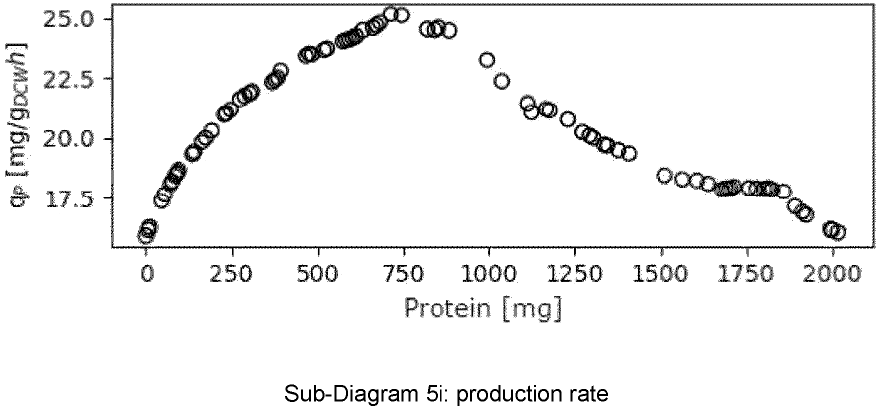

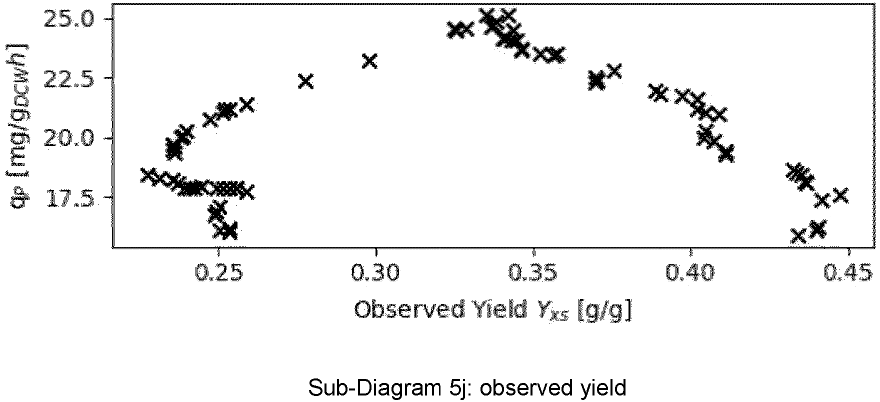

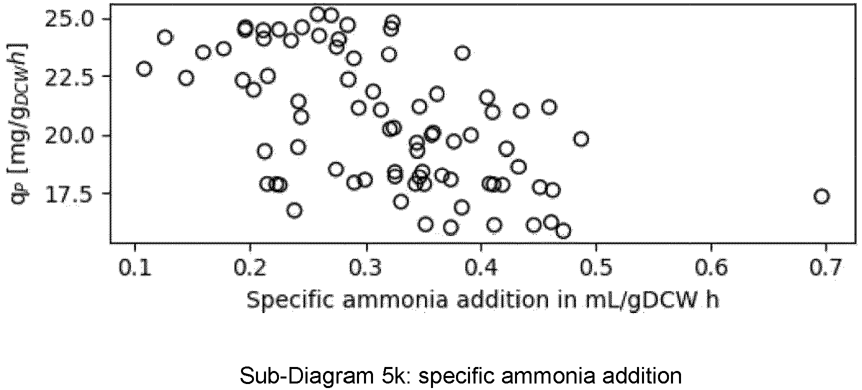

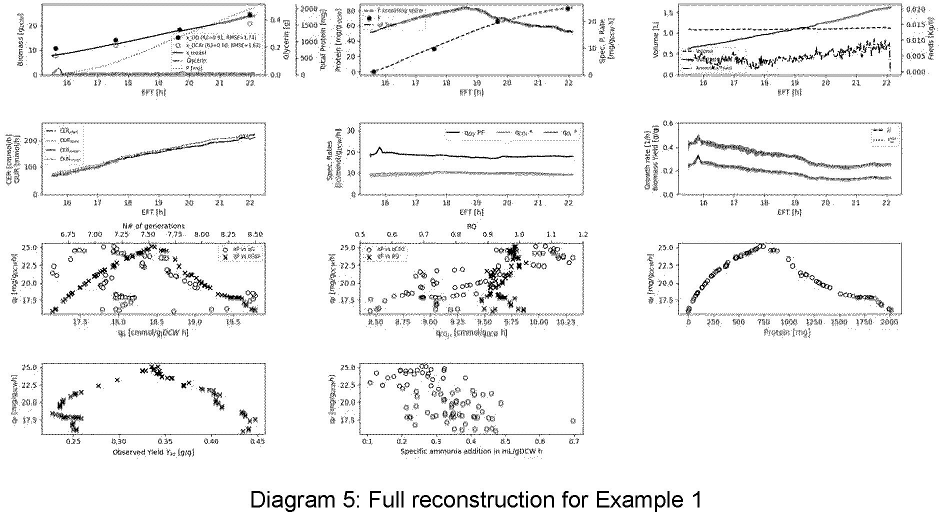

### Example 2:

Example 2 is based on example 1. Thus, everything of example 1 also applies to example 2 except from the fact that example 2 refers to multiple available fed-batch fermentations wherein the start of the analysis is always the beginning of induction. In this case a full characterization about the interaction between protein content qP, the state variables and the specific rates of the training data set can be presented. The substrate S is Glycerol in the present example and the substrate uptake rate is disclosed as qG. The features Z of the feature matrix F can be extracted and visualization of key features Z and their interactions are presented in Diagram 6.

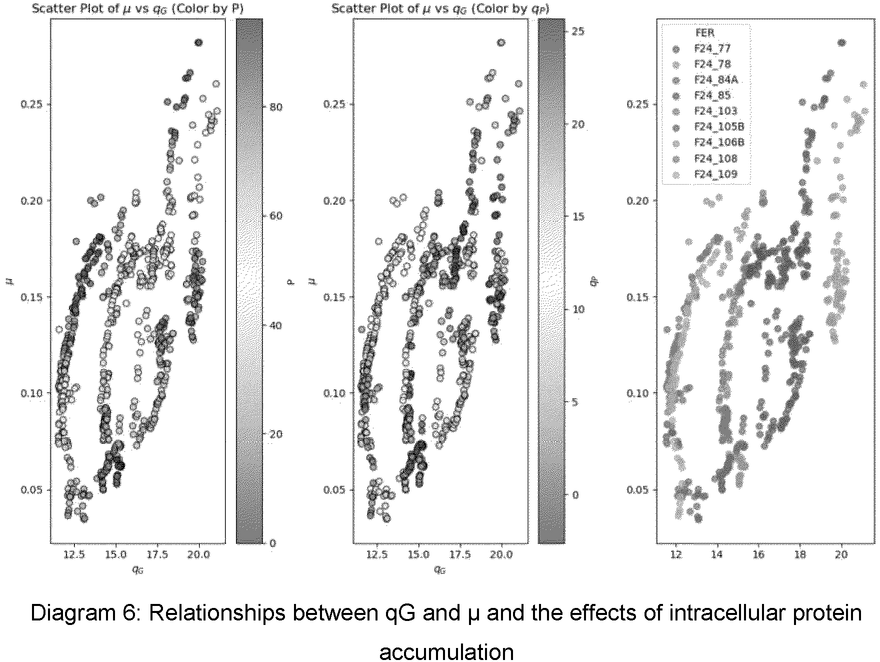

### Example 3:

Example 3 is similar to example 1. However, the feature matrix F is composed by the following features Z: the substrate specific uptake rate qG, the biomass specific growth rate µ, the biomass specific carbon dioxide generation rate q_{CO2}, the biomass specific oxygen uptake rate q_{O2},the number of generations of the culture, the intracellular protein content P, the biomass yield on substrate Yₓₛ, and the respiratory quotient R_{Q}. A Principal Component Analysis was performed over the training data-set in the matrix F to determine the minimal number of linearly independent components are required to explain the variability in the data. Diagram 7 shows that there are three main components that are required to explain more than 95% of the variability in the data. This information can be used by anyone skilled in the art of data science and bioprocess digitalization to reduce the space, number of features required to provide a robust dataset for qP regression.

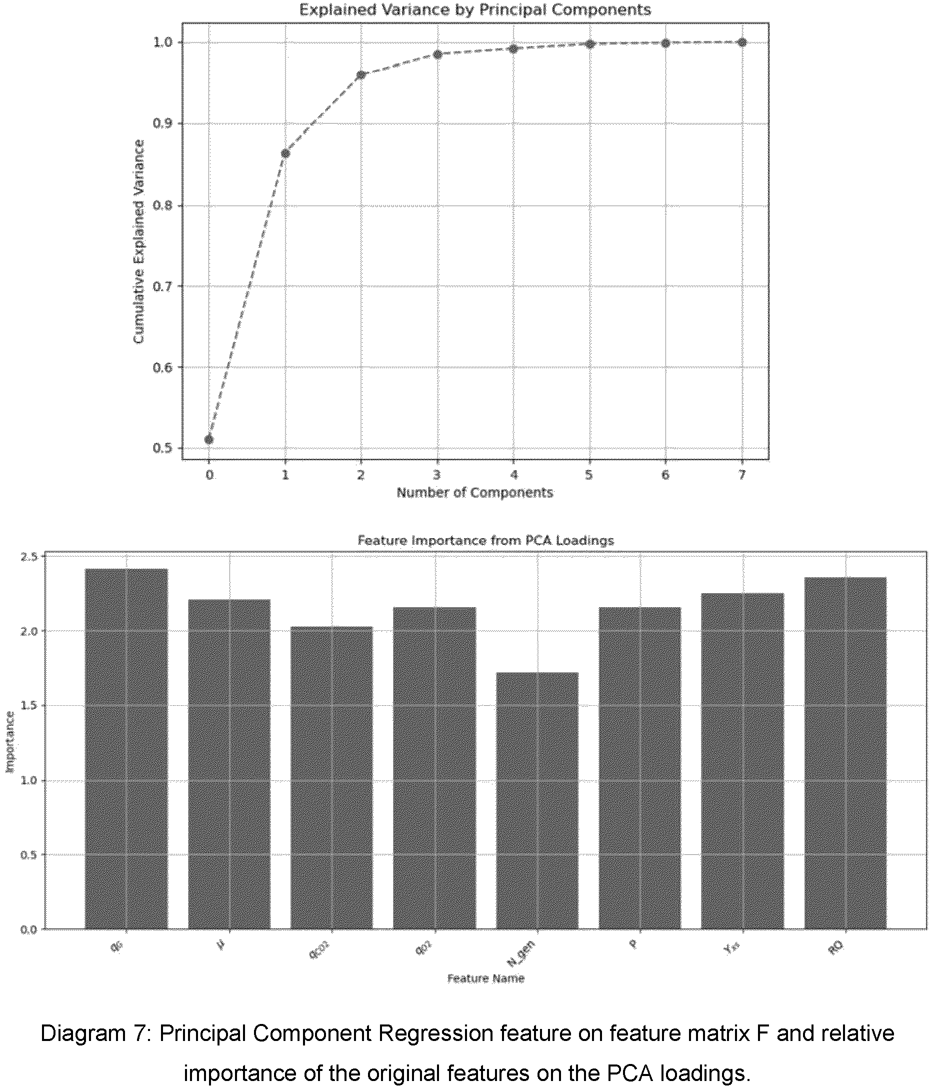

In this example, the entire dataset is regressed against qP using three algorithms in the field of supervised learning: Partial Least Squares (PLS), Gaussian Process Regression (GPR), Multi Layered-Perceptron (MLP) and a min-max scaled MLP of the feature matrix F. Table 2 shows that PLS showed the lowest fitting of the data with an R2 of 0.38 whereas the neural network (MLP and MLP-min-max) showed the highest R2 of 0.77 and 0.94 respectively. The biplots of qP estimated and qP observed are presented in Diagram 8 for PLS regression, Diagram 9 for GPR regression, Figure 10 for MLP regression and 11 for the min-max scaled X MLP regression.

**Table 2: Comparison of the performance of supervised learning methods for estimation of qP.**

| Algorithm | mean squared error | *R*² score |
|---|---|---|
| PLS | 28.03 | 0.38 |
| GPR | 9.77 | 0.78 |
| MLP | 10.10 | 0.77 |
| MLP-minmax | 2.7 | 0.94 |

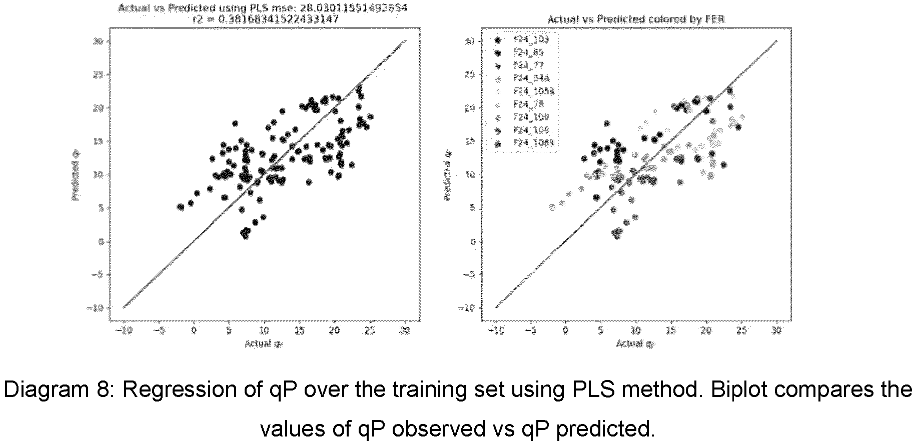

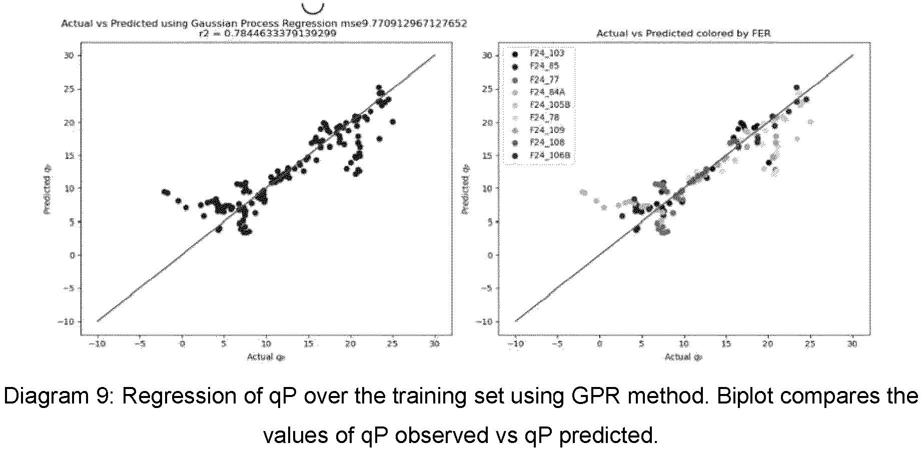

### Example 4:

In example 4 the particle filter is integrated from the beginning of the induction phase, until the current fermentation time, without prior knowledge about the protein content. The only information used in the beginning by the mathematical model is a range of the initial concentrations of the state vector, and a prior probability for the specific growth and substrate consumption rates.

In this deployment stage, the offline substrate S, offline biomass X and offline protein P concentrations are not known during the fermentation time. The reconstructed fermentation profile is shown in Diagram 12. Diagram 12 consists of ten Sub-Diagrams 12a to 12j.

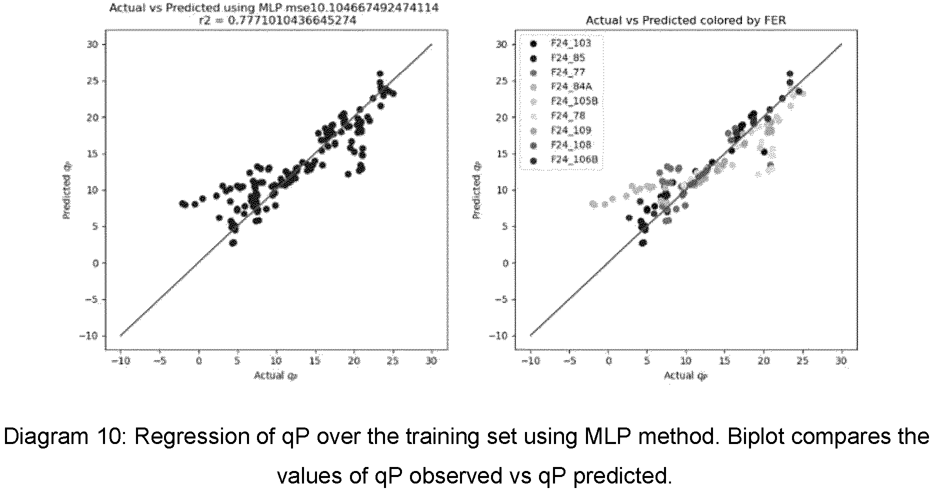

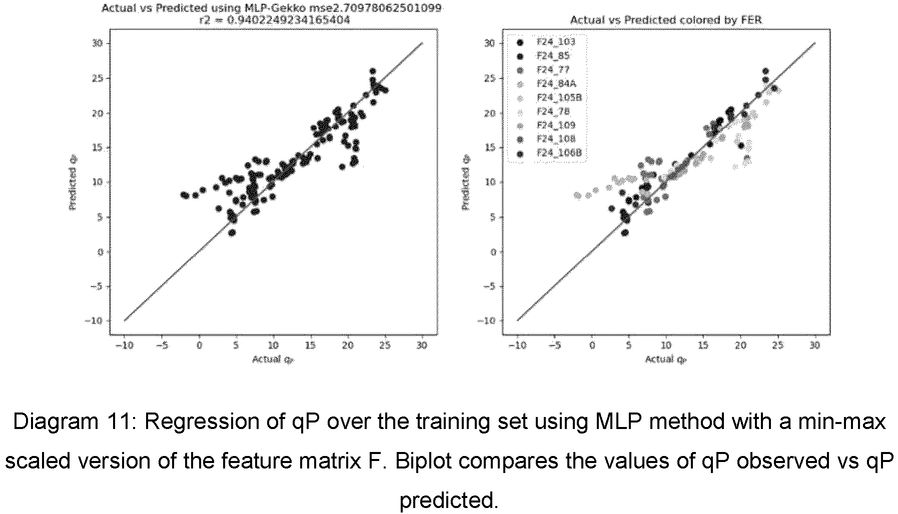

The first row of Diagram 12 shows data referring to Biomass X, Protein P and the volume V' of the bioreactor as offline data as measured and as estimated (Sub-diagrams 12a-12c). The second row of Diagram 12 shows CER, specific rates (protein production rate qP and substrate uptake rate qS/qG) and growth rates as online data and augmented times series data as measured and estimated (Sub-diagrams 12d-12f). The third and forth row each shows the protein production rate over time in different diagrams as measured and as expected ((Sub-diagrams 12g-12j). In the following the Sub-diagrams are disclosed, in order to be able to better visualise the content.

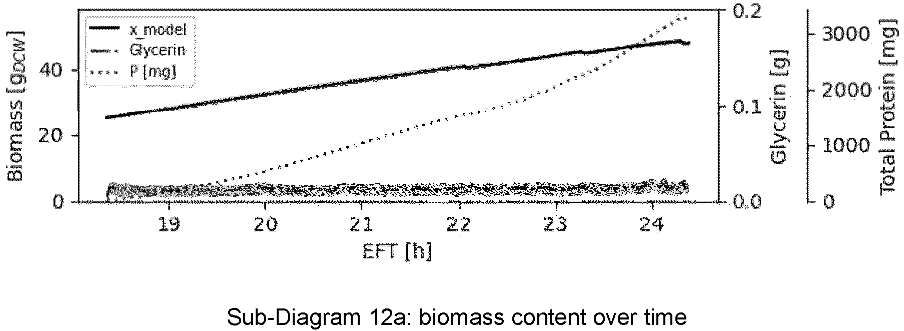

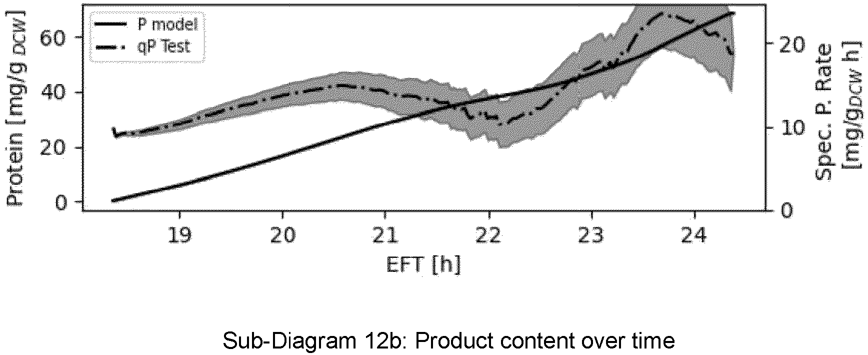

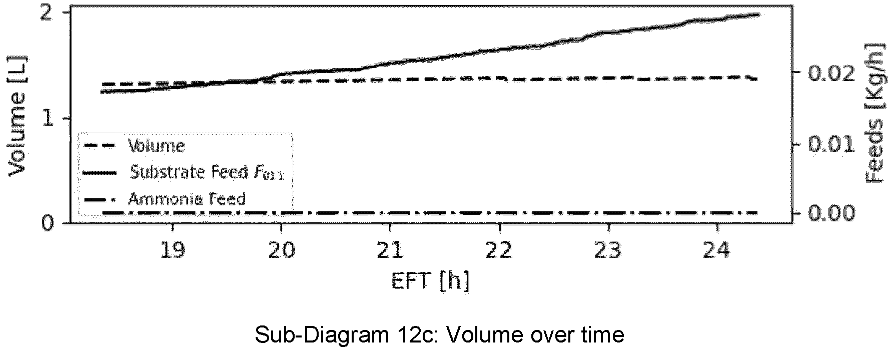

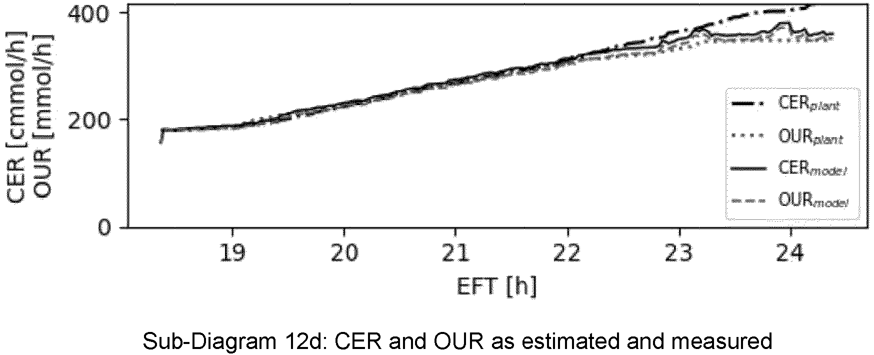

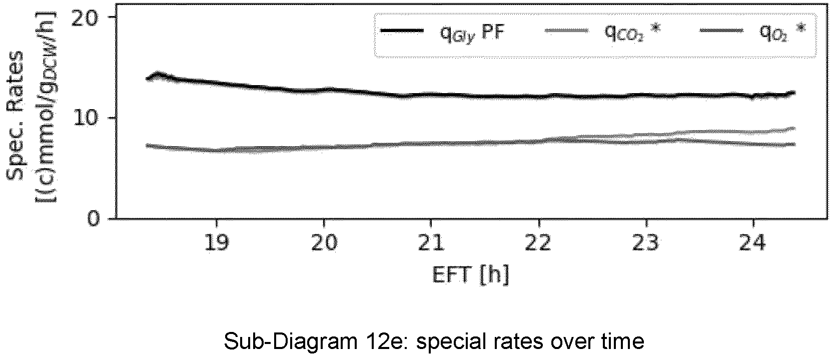

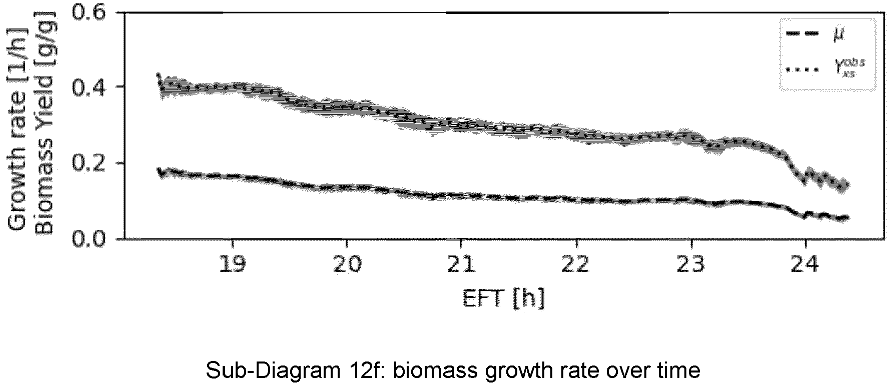

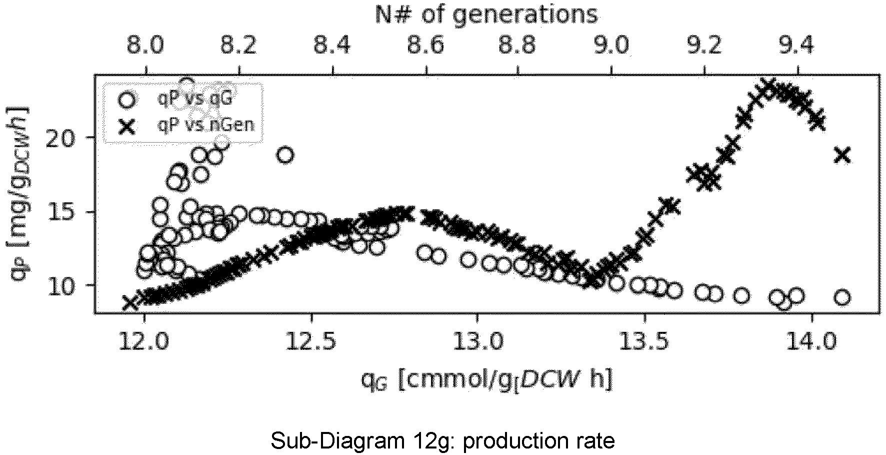

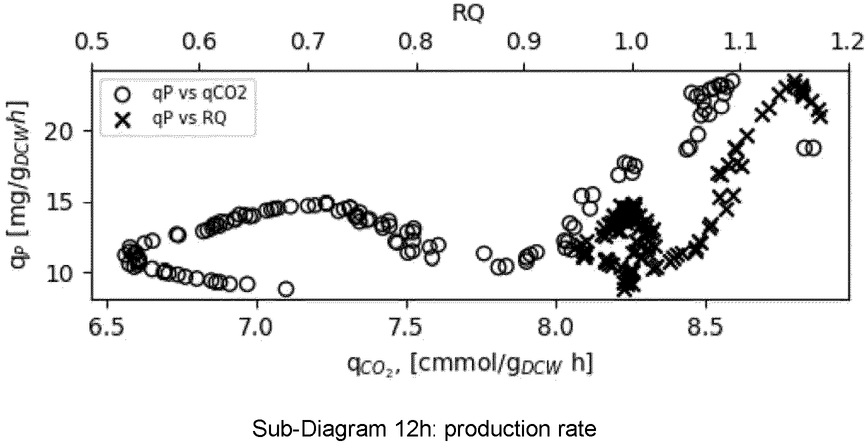

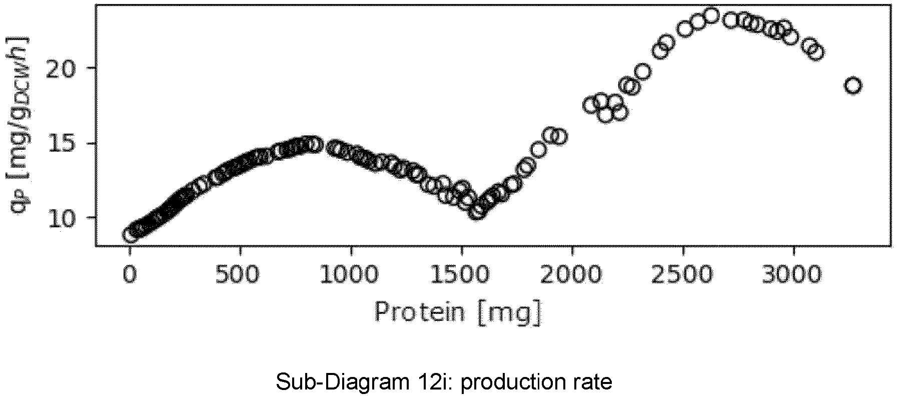

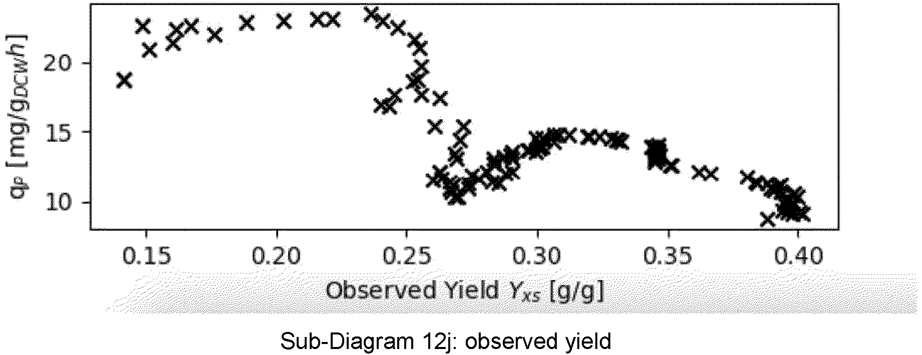

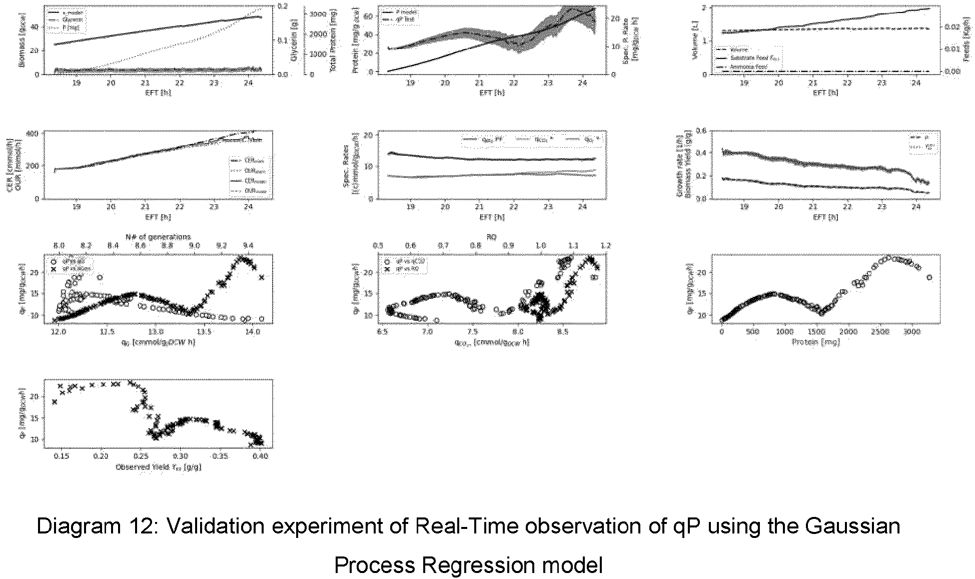

### Example 5:

Example 5 is similar to example 4. The reconstructed fermentation profile is shown in Diagram 15 but this time using MLP model regression instead. Posterior knowledge of the offline protein and evaluation on the qP is added to this fermentation wherein the regression quality over the measured offline protein data is compared in Diagram 13 and shows a quality of regression over 97%. In context, the qP model regression shows a good agreement with the offline biomass measurements and also support of the carbon-limiting nature of the induction stage, as shown in Figure 14. Fully demonstrating the potential of the invention to allow real time estimation of qP and protein values in recombinant cultured of β-casein producing strains of *E. coli.*

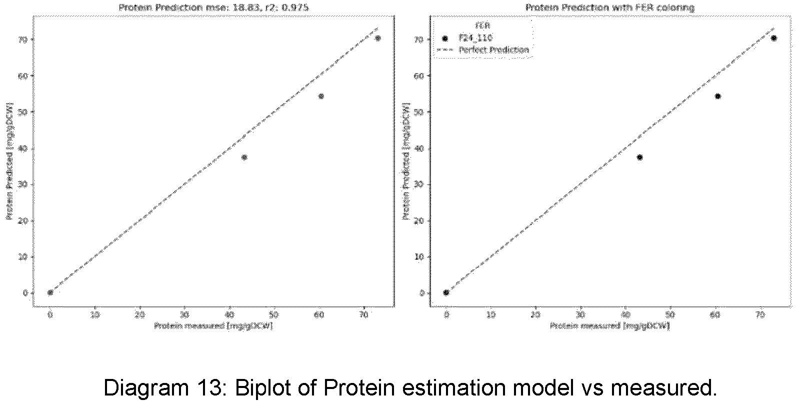

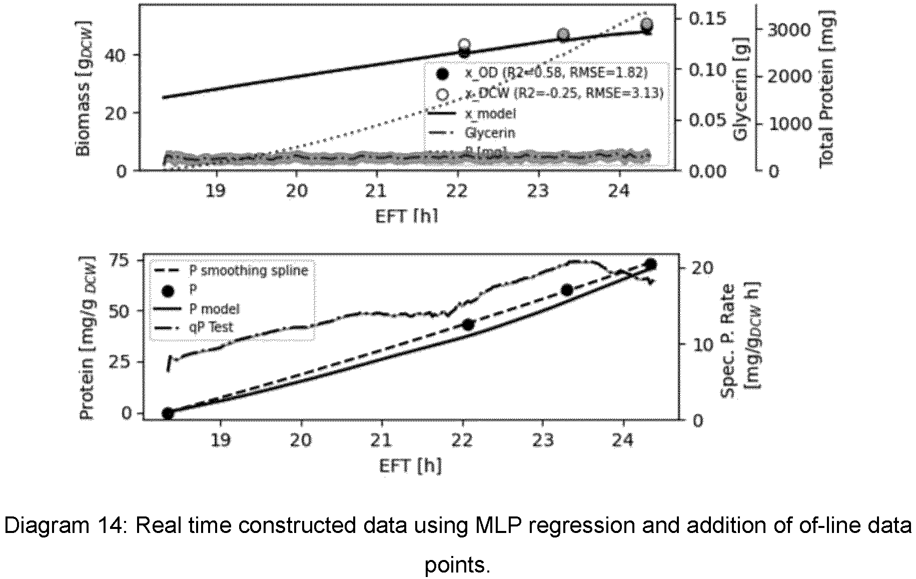

Diagram 15 consists of ten Sub-Diagrams 15a to 15j.The first row of Diagram 15 shows data referring to Biomass X, Protein P, Substrate G and the volume V of the bioreactor as offline data as measured and as estimated (Sub-Diagrams 15a-15c). The second row of Diagram 15 shows CER, specific rates (protein production rate qP and substrate uptake rate qS/qG) and growth rates as online data and augmented times series data as measured and estimated (Sub-Diagrams 15d-15f). The third and forth row each shows the protein production rate over time in different diagrams as measured and as expected (Sub-Diagrams 15g-15j). In the following the subdiagramms are disclosed, in order to be able to better visualise the content.

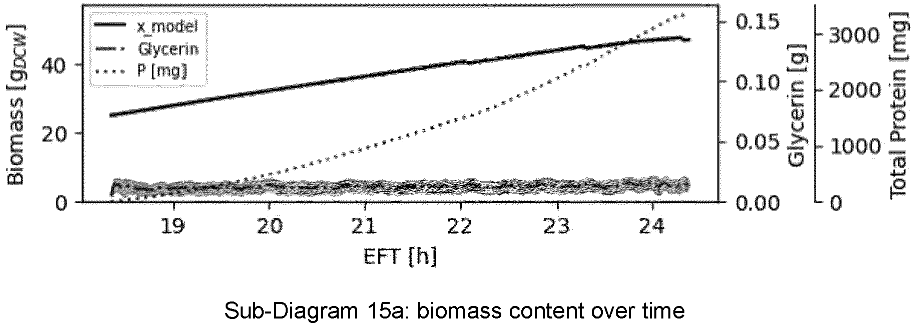

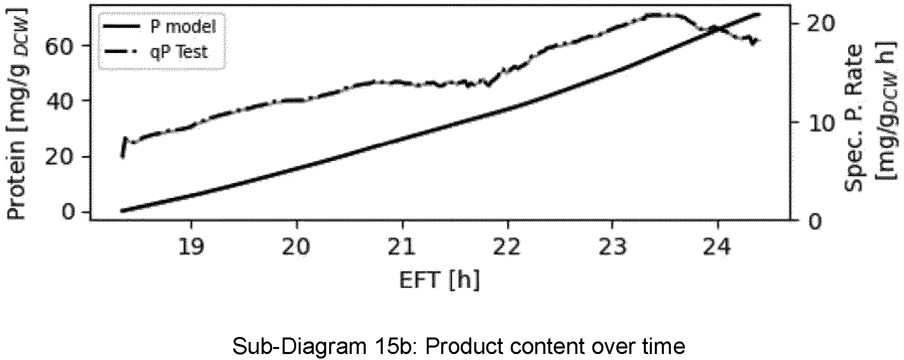

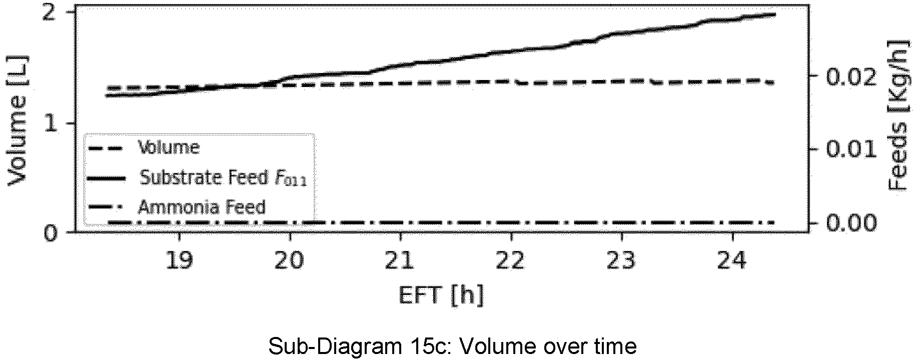

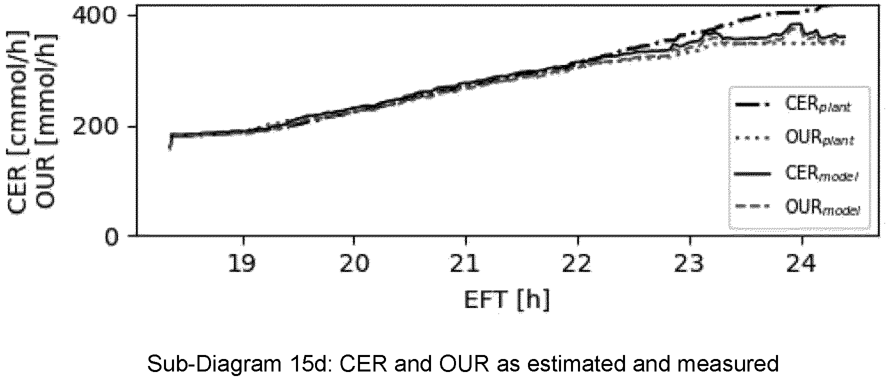

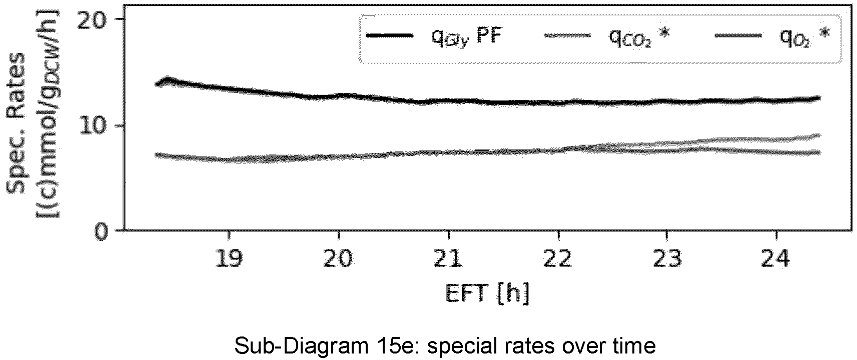

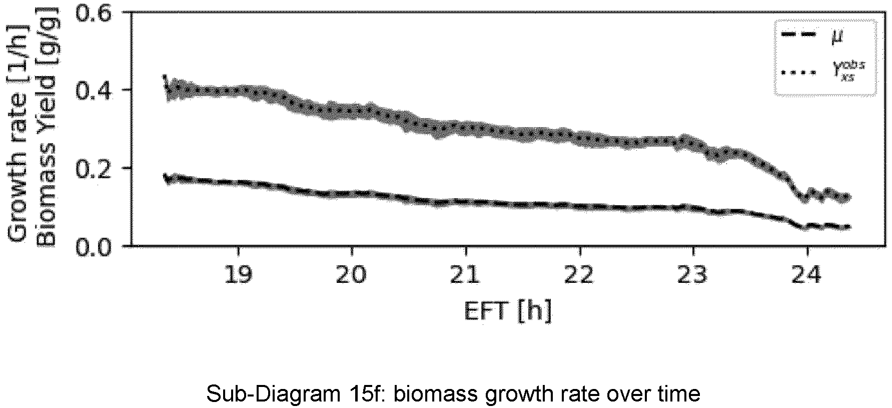

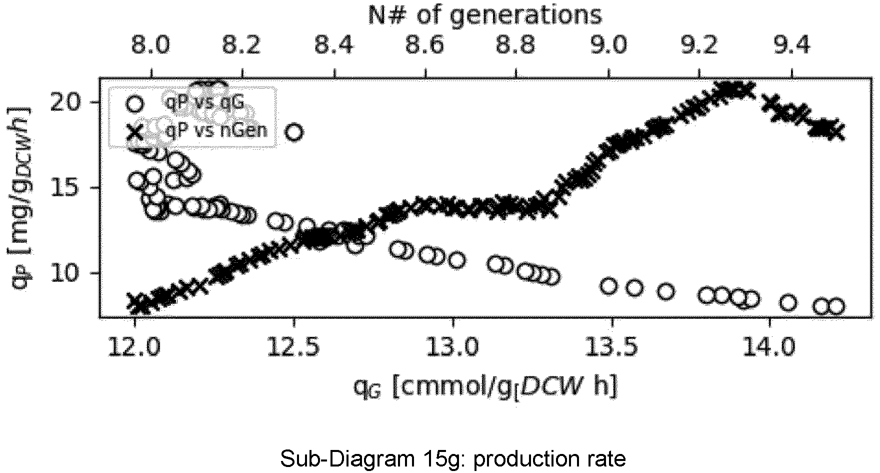

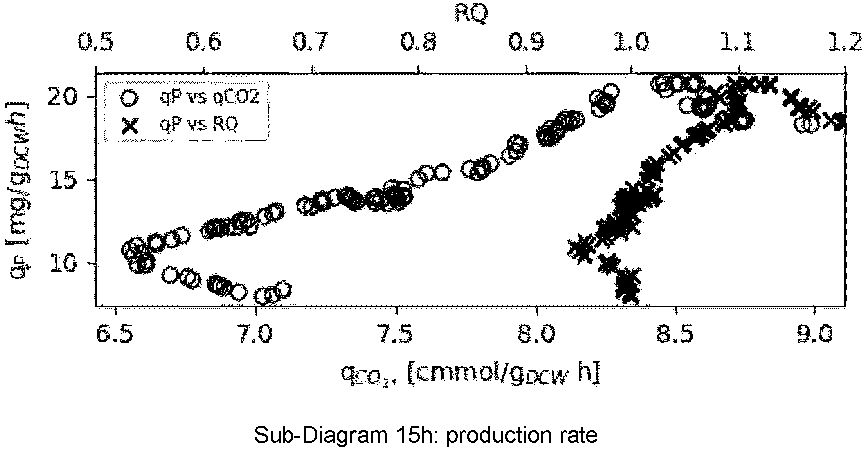

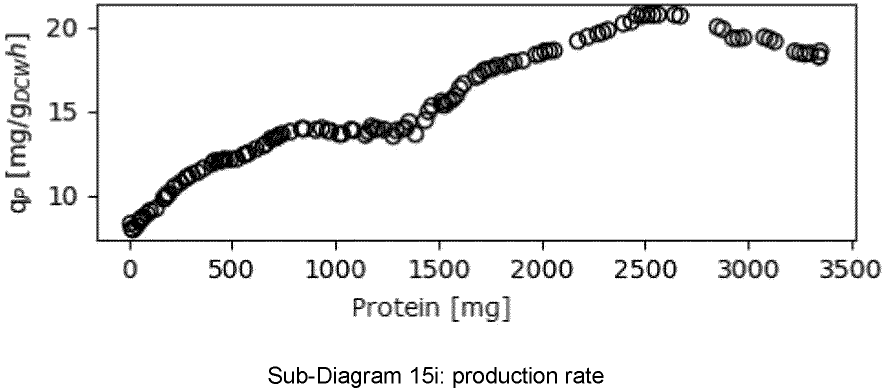

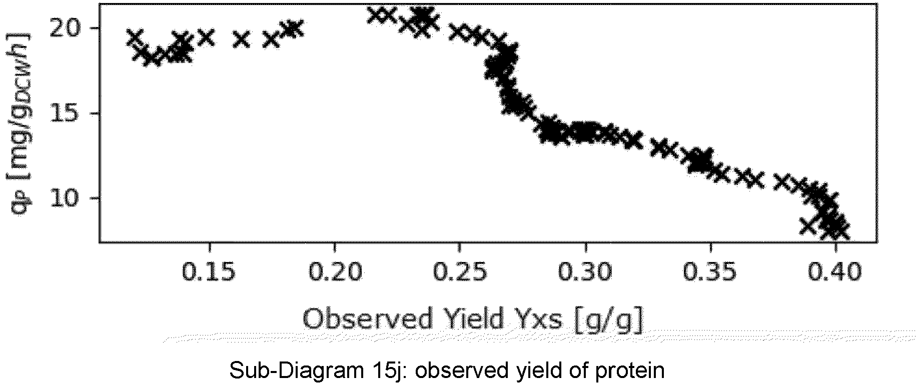

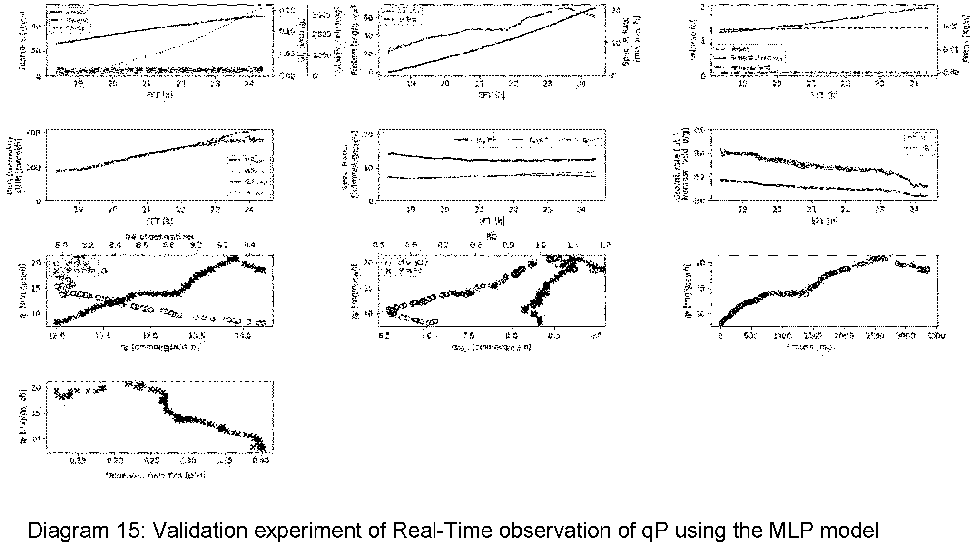

### BRIEF DESCRIPTION OF THE FIGURES

The invention will be explained in closer detail by reference to a preferred embodiment, which is depicted schematically in the figures.
Fig. 1 shows a schematic diagram of the system according to the present invention comprising a bioreactor with a computing unit capable of conducting the mathematical method.
Fig. 2 shows diagrams which indicate the perfect point of time to end the fermentation and obtain the β-casein produced.
Fig. 3 shows diagrams which indicate the a point of time when the perfect time to end the fermentation and obtain the β-casein produced was missed

### WAYS TO IMPLEMENT THE INVENTION

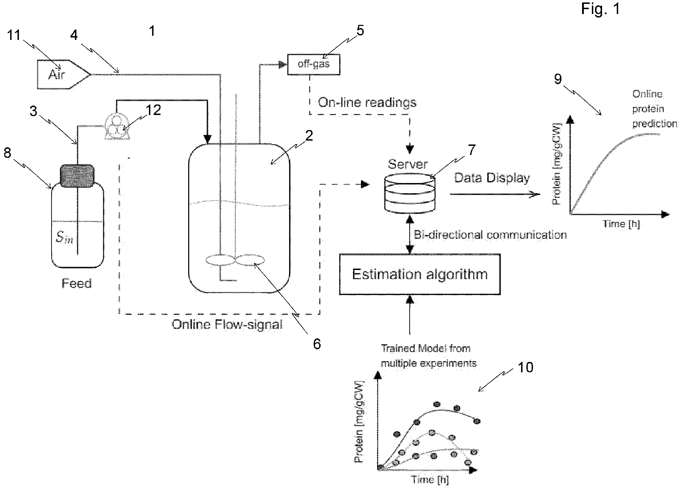

Fig. 1 shows a schematic diagram of the system 1 according to the present invention comprising a bioreactor 2 with a computing unit 7 capable of conducting the mathematical method according to the present invention.

In the present embodiment the bioreactor 2 comprises at least one culture of protein producing *Escherichia coli* bacteria for performing a fed-batch fermentation process in Glycerol as substrate S in order to produce β-casein as wished protein. The bioreactor 2 comprises an agitator 6 to stir the substrate S to ensure proper mixing.

The bioreactor 2 further comprises an inlet pipe 3 for feeding Glycerol into the bioreactor 2 to obtain an adequate medium for the protein producing microorganisms. In the present embodiment the substrate S is contained and stored in a substrate container 8. The substrate container 8 is connected to one end of the inlet pipe 3. The other end of the inlet pipe 3 is connected to the bioreactor 2 so that substrate S can be fed from the substrate container 8 into the bioreactor 2. To be able to supply substrate S to the bioreactor 2 the inlet pipe 3 is equipped with a pump 12 which pumps the substrate S to the bioreactor 2, if required. A second inlet pipe 4 is comprised to be able to feed gas into the bioreactor 2. In the present embodiment the gas is O₂ from a gas container 11 as *Escherichia coli* bacteria are aerobe bacteria which need an adequate amount of O₂ in the media to survive, grow and produce protein P without being stressed out. An outlet pipe 5 for exhaust air from the bioreactor 2 and an off-gas analyzer (not shown) which is connected to the outlet-pipe 5 to measure the gas content, in particular the O₂ and CO₂ content according to the present embodiment of the exhaust air are further comprised. As the adequate O₂ content is essential for the bacteria a controlling unit (included in the computing unit 7) to control and adapt an oxygen level in the media of the bioreactor 2 is further comprised.

Additionally a mass-flow sensor (not shown) which is connected to the inlet pipe 4 to measure the mass-flow in the inlet pipe 4. Further a dissolved oxygen sensor (not shown), which measures the dissolved oxygen in the bioreactor 2. The off-gas analyzer, the mass-flow sensor and the dissolved oxygen sensor are used to obtain data, which are collected by the computing unit 7, which is adapted to execute the computer-implemented method. A softsensor in the computing unit 7 obtains data referring the O₂ percentage and the CO₂ percentage in the exhaust air and converts them into data referring to the O₂ consumption and the CO₂ production.

Different data are obtained, namely offline data, which are obtained from the bioreactor 2 via manually or automatically taking samples and online data which are the data that are converted via softsensor to obtain different macroscopic states and rates data.

The obtained data are then combined and stored to obtain and improve a training data set in the computer unit 7. The obtained training data set is used to create a first feature matrix F, whereby initially unknown values of the feature matrix F are completed over time in course of the biotechnological process. The more data are filled in the feature matrix F, the more accurate is the outcome. Afterwards the training data set is augmented by processing the training data set through a mathematical model to receive estimations of specific rates, states and concentrations in the form of a time-series data, wherein equations to describe and solve the rate transfer of carbons and electrons under the assumption of pseudo-steady state and equations to describe the development of global mass balance and total protein content over time are used by the mathematical model (bidirectional communication). In the following a probability hypothesis (estimation algorithm) is generated and validated, of how the data of the training data set is interconnected, so that an augmented time-series data for the fermentation according to the present embodiment of the invention, is produced. The estimated states, rates and concentrations are displayed as curves in a diagram 9.

When training the mathematical model with a machine learning method, using the augmented time-series data to estimate the protein production rate qP, at least one feature Z of the augmented time-series data is regressed against at least one of the obtained offline data of the bioreactor 2. Finally the regressed time series data is used to estimate the protein production rate qP at any point of time of the current fermentation. The estimated protein production rate qP is displayed as a second diagram 10.

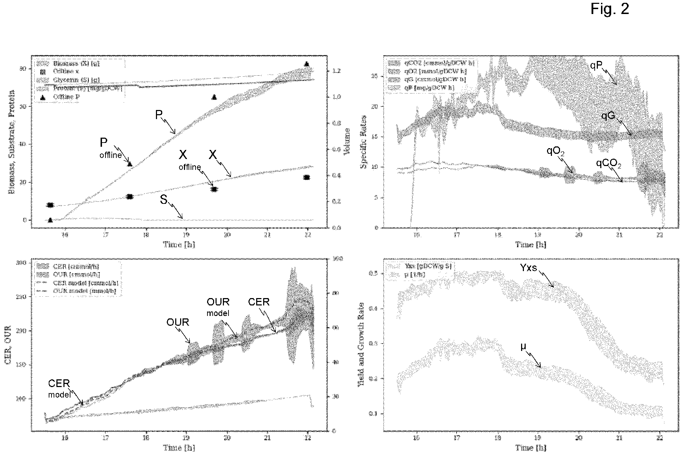

Fig. 2 shows four diagrams. The first diagram refers the model states biomass X, substrate S and Protein P. Protein P is in the present embodiment β-casein. The data for P, S and µ curve show the estimations made by the mathematical model. Further data for samples taken from the bioreactor for offline data Poff and Xoff are shown, which very well correspond to the estimated data. The second diagram below the first diagram shows CER rate and OUR rate which are converted online data. Additionally the CER and OUR rate as estimated by the mathematical model as dashed line each. Also referring the online data it is shown that the estimated and the measured data correspond very well. The third diagram next to the first diagram indicates specific rates, which result from the augmented training data set, that was obtained by storing and combining the online and offline data. The forth diagram finally shows the estimated protein production rate qP of β-casein. The curves of the diagrams indicate that the optimal point in time to end the fermentation and obtain the protein p produced. The curves are still increasing at that point, however no longer very much.

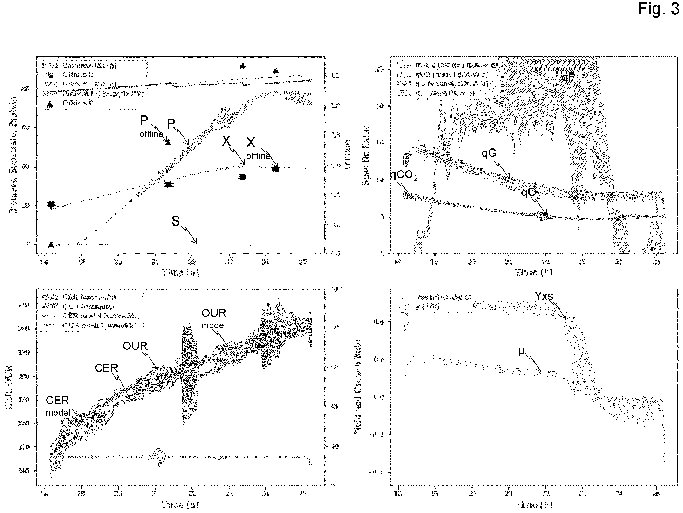

Fig. 3 shows four diagrams. The first diagram refers the model states biomass X, substrate S and Protein P. Protein P is in the present embodiment β-casein. The data for P, S and µ curve show the estimations made by the mathematical model. Further data for samples taken from the bioreactor for offline data Poff and Xoff are shown, which very well correspond to the estimated data. The second diagram below the first diagram shows CER rate and OUR rate which are converted online data. The third diagram next to the first diagram indicates specific rates, which result from the augmented training data set, that was obtained by storing and combining the online and offline data. Additionally the CER and OUR rate as estimated by the mathematical model as dashed line each. Also referring the online data it is shown that the estimated and the measured data correspond very well.The forth diagram finally show the estimated protein production rate qP of β-casein. The curves of the diagrams indicate that the optimal point in time to end the fermentation and obtain the protein P produced has been missed. The curves are already decreasing at that point.

### LIST OF REFERENCE SIGNS

- 1: System
- 2: Bioreactor
- 3: first inlet pipe
- 4: second inlet pipe
- 5: outlet pipe
- 6: agitator
- 7: computing unit
- 8: substrate container
- 9: first diagram
- 10: second diagram
- 11: gas feed
- 12: pump
- X: Biomass
- F: Feature Matrix
- Z: Feature Variable
- V: Volume of the bioreactor
- S: Substrate
- P: Product
- G: Glycerol (Substrate)
- µ: growth rate of biomass
- qP: protein production rate
- qS: substrate uptake rate
- qG: Glycerol (substrate) uptake rate

### LIST OF CITED REFERENCE

[1] Arshpreet Bhatwa et al. "Challenges Associated With the Formation of Recombinant Protein Inclusion Bodies in Escherichia coli and Strategies to Address Them for Industrial Applications". In: Frontiers in Bioengineering and Biotechnology 9 (Feb. 10, 2021), p. 630551. issn: 2296-4185. doi: 10.3389/fbioe.2021.630551. url: https: //www.frontiersin.org/articles/10.3389/fbioe.2021.630551/full (visited on 05/07/2024).
[2] Matthew P. DeLisa et al. "Monitoring GFP-operon fusion protein expression during High cell density cultivation ofEscherichia coli using an on-line optical sensor". In: Biotechnology and Bioengineering 65.1 (Oct. 5, 1999), pp. 54-64. issn: 0006-3592, 1097-0290. doi: 10.1002/(SICI)1097-0290(19991005)65:1<54::AID-BIT7>3.0.CO;2-R. url: https://onlinelibrary.wiley.com/doi/10.1 002/(SICI)1097-0290(19991005)65: 1%3C54::AID-BIT7%3E3.0.CO;2-R (visited on 05/08/2024).
[3] Kasper Hettinga and Etske Bijl. "Can recombinant milk proteins replace those produced by animals?" In: Current Opinion in Biotechnology 75 (June 2022), p. 102690. issn: 09581669. doi: 10.1016/j.copbio.2022.102690. url: https://linkinghub. elsevier.com/retrieve/pii/S0958166922000106 (visited on 05/09/2024).
[4] Chung-Jr Huang, Henry Lin, and Xiaoming Yang. "Industrial production of recombinant therapeutics in Escherichia coli and its recent advancements". In: Journal of Industrial Microbiology and Biotechnology 39.3 (Mar. 1, 2012), pp. 383-399. issn: 1476-5535, 1367-5435. doi: 10.1007/s10295-011-1082-9. url: https://academic.oup.com/jimb/ article/39/3/383/5994493 (visited on 05/07/2024).
[5] Stefan Kittler et al. "Cascaded processing enables continuous upstream processing with E. coli BL21(DE3)". In: Scientifc Reports 11.1 (June 1, 2021), p. 11477. issn: 2045-2322. doi: 10.1038/s41598- 021- 90899- 9. url: https://www.nature.com/articles/ s41598-021-90899-9 (visited on 05/07/2024).
[6] Julian Kopp et al. "Repetitive Fed-Batch: A Promising Process Mode for Biomanufacturing With E. coli". In: Frontiers in Bioengineering and Biotechnology 8 (Nov. 10, 2020), p. 573607. issn: 2296-4185. doi: 10.3389/fbioe.2020.573607. url: https://www.frontiersin.org/articles/10.3389/fbioe.2020.573607/full (visited on 05/07/2024).
[7] M.B. Nielsen, A.S. Meyer, and J. Arnau. "The Next Food Revolution Is Here: Recombinant Microbial Production of Milk and Egg Proteins by Precision Fermentation". In:Annual Review ofFood Science and Technology (2023). Publisher: Annual Reviews. issn:1941-1413. doi: https://doi.org/10.1146/annurev-food-072023-034256. url: https://www.annualreviews.org/content/journals/10.1146/annurev- food-072023-034256.
[8] John Puetz and Florian M. Wurm. "Recombinant Proteins for Industrial versus Pharmaceutical Purposes: A Review of Process and Pricing". In: Processes 7.8 (July 24, 2019), p. 476. issn: 2227-9717. doi: 10.3390/pr7080476. url: https://www.mdpi.com/2227- 9717/7/8/476 (visited on 05/09/2024).
[9] Henning Steinfeld et al. Livestock's long shadow: environmental issues and options. Food\& Agriculture Org., 2006.
[10] Zi-Xu Zhang et al. "Strategies for efcient production of recombinant proteins in Escherichia coli: alleviating the host burden and enhancing protein activity". In: Microbial Cell Factories 21.1 (Sept. 15, 2022), p. 191. issn: 1475-2859. doi: 10.1186/s 12934-022- 01917-y. url: https://microbialcellfactories.biomedcentral.com/articles/ 10.1186/s12934-022-01917-y (visited on 05/07/2024).

## Claims

1. A computer-implemented method for estimating a specific protein production rate (qP) of at least one culture of protein (P) producing microorganisms in a biotechnological process, in particular a fermentation process, in a bioreactor (2), comprising at least the steps of
- obtaining data from the bioreactor (2) by measuring data and converting the measured data directly into macroscopic reaction rates to obtain online data, and/or by taking samples at least once manually or automatically from the bioreactor (2) to obtain offline data,
- combining and storing the online and/or offline data to obtain and improve a training data set;
- augmenting said training data set by processing the training data set through a mathematical model to receive estimations of specific rates, states and concentrations in the form of a time-series data,
wherein equations to describe and solve the rate transfer of carbons and electrons under the assumption of pseudo-steady state and equations to describe the development of global mass balance and total protein content over time are used by the mathematical model,
wherein a probability hypothesis is generated and validated, of how the data of the training data set is interconnected, so that an augmented time-series data for the current biotechnological process, in particular the fermentation process, is produced,
- training the mathematical model with a machine learning method, using the augmented time-series data to estimate the protein production rate (qP), wherein at least one feature (Z) of the augmented time-series data is regressed against at least one of the obtained offline data of the bioreactor (2),
and the regressed time series data is used to estimate the protein production rate (qP) at any point of time of the current biotechnological process, in particular the fermentation process.

2. The computer-implemented method according to claim 1, wherein a softsensor obtains the online data referring to the percentage of oxygen O₂ and carbon dioxide CO₂ in the exhaust air of the bioreactor (2) and converts the obtained online data into converted online data which refer to the O₂ consumption and CO₂ production in the bioreactor (2).

3. The computer-implemented method according to claim 1 or 2, wherein the obtained online data are at least one selected from the group comprising Carbon Evolution Rate (CER), Oxygen Uptake Rate (OUR), ammonia uptake rate, feed rates, pH, temperature and aeration.

4. The computer-implemented method according to any one of the preceding claims, wherein the obtained offline data are at least one selected from the group comprising biomass concentration and/or mass, substrate concentration and/or mass, total protein content concentration and/or mass and/or the biomass specific concentration of protein inside the cell.

5. The computer-implemented method according to any one of the preceding claims, wherein the mathematical model uses model constants, wherein the only model constants of the mathematical model that are tunable and subject to further optimization are the biomass molar mass (mwₓ), the biomass maintenance coefficient (mₑ) and the biomass degree of reduction (γ_{X}), so that the mathematical model can be adapted for any unknown microorganisms.

6. The computer-implemented method according to any one of the preceding claims, wherein the mathematical model uses model states such as the mass of biomass (X), the mass of substrate (S), the relative mass of intracellular protein (P) and the volume of the reactor (V), which model states come from previous experience and input, in order to estimate the specific protein production rate (qP).

7. The computer-implemented method according to claim 6, wherein the model states are initially unknown, but the probability hypothesis (h(x,θ)) is formulated for every possible combination of values for initial model states (x0), such as mass of biomass (X), the mass of substrate (S), the relative mass of intracellular protein (P) and the volume of the reactor (V), and the parameters (θ_0), such as the substrate uptake rate (qS) and growth rate (q*µ*).

8. The computer-implemented method according to claim 7, wherein the evaluation of the probability of the true state of the bioreactor (2) to the obtained offline data is based on sequential Monte Carlo method.

9. The computer-implemented method according to claim 8, wherein the estimation of the protein production rate qP at any point in time is formulated as *q̂_{P} = dP̂(t)*/*dt* + *µP̂*, wherein *P̂*(*t*) is the intracellular protein in time, preferably obtained as a smoothing spline function, d*P̂*(t)/dt is the rate of change of the intracellular protein in time and µ*P̂* is the product of the intracellular protein at a specific time and the growth rate (qµ) obtained after evaluating the probability hypothesis.

10. The computer-implemented method according to any one of the preceding claims, wherein the estimated value of the production rate (qP) is formulated as a direct interaction between a smoothing spline function for the intracellular protein (P) in time and the growth rate (qµ) for each time determined.

11. The computer-implemented method according to any one of the preceding claims, wherein the estimated production rate (qP) is used to estimate the protein production and content.

12. The computer-implemented method according to any of the preceding claims, wherein the augmented time series data is produced via numerical regression.

13. System (1) for performing a computer-implemented method according to any of claims 1 to 12 at least comprising
- a bioreactor (2) for performing a biotechnological process, in particular a fermentation process, comprising at least one culture of protein producing microorganisms,
- an inlet pipe (3) for feeding at least one fluid into the bioreactor (2) to obtain an adequate medium for the protein producing microorganisms,
- an outlet pipe (5) for exhaust air from the bioreactor (2),
- an off-gas analyzer which is connected to the outlet-pipe (5) to measure the gas content of the exhaust air,
- a mass-flow sensor which is connected to the inlet pipe (3) to measure the mass-flow in the inlet pipe (3),
- a dissolved oxygen sensor, which measures the dissolved oxygen in the bioreactor (2),
- a controlling unit (7) to control and adapt an oxygen level in the medium of the bioreactor (2);
- a computing unit (6) which is adapted to execute the computer-implemented method according to any of claims 1 to 12.

14. System according to claim 13, wherein online and offline data are obtained by measuring and converting data from the outlet pipe (5).

15. Computer program product comprising instructions to cause a system (1) for performing a computer-implemented method according to any of claims 1 to 12 to perform all the steps of said computer-implemented method.
